# EUROPEAN PATENT SPECIFICATION

(11) **EP 4 294 951 B1**
(45) Date of publication and mention of the grant of the patent: **27.11.2024**
(21) Application number: 23725990.8
(22) Date of filing: 09.05.2023
(51) Int. Cl.: C12Q 1/6886

(54) **NOVEL RNA MOLECULE FOR CANCER DETECTION**
NEUARTIGES RNA-MOLEKÜL FÜR DEN NACHWEIS VON KREBS
NOUVELLE MOLÉCULE D'ARN POUR LA DÉTECTION DU CANCER

(30) Priority: 13.05.2022 EP 22173271
(43) Date of publication of application: 27.12.2023
(73) Proprietor: Hummingbird Diagnostics GmbH, 69120 Heidelberg (DE)
(72) Inventor: STEINKRAUS, Bruno, 21271 Hanstedt (DE); SIKOSEK, Tobias, 69117 Heidelberg (DE); HOROS, Ratislav, 69126 Heidelberg (DE)
(74) Representative: Geling, Andrea
(86) International application number: PCT/EP2023/062195
(87) International publication number: WO 2023/217735

(56) References cited:
- WO-A1-2018/119421
- WO-A2-02/086443
- WO-A2-2016/065349
- SAVELYEVA ANNA V. ET AL: "Variety of RNAs in Peripheral Blood Cells, Plasma, and Plasma Fractions", BIOMED RESEARCH INTERNATIONAL, vol. 2017, 1 January 2017 (2017-01-01), pages 1 - 10, XP055872794, ISSN: 2314-6133, DOI: 10.1155/2017/7404912
- HA SHANSHAN ET AL: "Reduced ribosomal RNA expression and unchanged ribosomal DNA promoter methylation in oral squamous cell carcinoma", MOLECULAR GENETICS & GENOMIC MEDICINE, vol. 7, no. 7, 1 July 2019 (2019-07-01), XP093065011, ISSN: 2324-9269, DOI: 10.1002/mgg3.783
- UEMURA M ET AL: "Overexpression of ribosomal RNA in prostate cancer is common but not linked to rDNA promoter hypomethylation", ONCOGENE, vol. 31, no. 10, 8 August 2011 (2011-08-08), London, pages 1254 - 1263, XP093064961, ISSN: 0950-9232, DOI: 10.1038/onc.2011.319
- GONG Y ET AL: "Comparison of glyceraldehyde-3-phosphate dehydrogenase and 28S-ribosomal RNA gene expression in human hepatocellular carcinoma", HEPATOLOGY, vol. 23, no. 4, 1 April 1996 (1996-04-01), US, pages 734 - 737, XP093065013, ISSN: 0270-9139, DOI: 10.1002/hep.510230413

## Description

The present invention relates to a method of diagnosing lung cancer in a patient. Further, the present invention relates to a method of monitoring the course of lung cancer in a patient. Furthermore, the present invention relates to a method of determining whether a patient responds to a therapeutic treatment of lung cancer. The present invention also relates to a method of determining the risk for a relapse of lung cancer in a patient. In addition, the present invention relates to a method of detecting a minimal residual disease in a patient having lung cancer. Moreover, the present invention relates to the use of a kit for carrying out these methods.

### BACKGROUND OF THE INVENTION

Cancer is the uncontrolled growth of abnormal cells anywhere in a body. The abnormal cells are termed cancer cells, malignant cells, or tumor cells. Cancer cells can proliferate uncontrollably and form a mass of cancer cells.

The most common types of cancer in males are lung cancer, prostate cancer, colorectal cancer, and stomach cancer. In females, the most common types are breast cancer, colorectal cancer, lung cancer, and cervical cancer.

Lung cancer or bronchogenic carcinoma refers to tumors originating in the lung parenchyma or within the bronchi. It is one of the leading causes of cancer-related deaths in Europe and in the United States. Since 1987, lung cancer has been responsible for more deaths in women than breast cancer. It is estimated that there are 225,000 new cases of lung cancer in the United States annually, and approximately 160,000 die because of lung cancer. It is interesting to note that lung cancer was a relatively rare disease at the beginning of the 20th century. Its dramatic rise in later decades is attributable primarily to the increase in smoking among both males and females.

Historically, markers such as age, performance status and disease stage have been used to risk-stratify cancer patients and guide therapeutic decisions. These parameters provide some useful information, but more sensitive markers are clearly needed. Molecular and genetic studies have identified several such markers, which appear to play critical roles in carcinogenesis and affect patient outcomes. However, their prognostic and predictive value for cancer medicine has been low so far.

WO 02/086443 A2 discloses methods of diagnosis of lung cancer, compositions and methods of screening for modulators of lung cancer.

The present inventors identified a 28S ribosomal RNA (rRNA) fragment which is upregulated in lung cancer patients compared to healthy controls. In particular, the present inventors found that the 28S rRNA fragment is significantly upregulated in lung cancer patients compared to healthy controls. In addition, the present inventors showed that the 28S rRNA fragment is expressed more highly with increasing tumor stage in a clinical trial on lung cancer patients. After lung cancer surgery, the present inventors found that the 28S rRNA fragment level returns to normal level. Said RNA molecule can, thus, be used as biomarker for lung cancer diagnosis, monitoring, relapse control, minimal disease detection, and surgical resection status determination.

### SUMMARY OF THE INVENTION

In a first aspect, the present invention relates to a method of diagnosing lung cancer in a patient comprising the step of:
determining the level of an RNA molecule in a blood sample from a patient, wherein the RNA molecule comprises a nucleotide sequence according to SEQ ID NO: 1.

In a second aspect, the present invention relates to a method of monitoring the course of lung cancer in a patient comprising the step of:
determining the level of an RNA molecule in a blood sample from a patient, wherein the RNA molecule comprises a nucleotide sequence according to SEQ ID NO: 1.

In a third aspect, the present invention relates to a method of determining whether a patient responds to a therapeutic treatment of lung cancer comprising the step of:
determining the level of an RNA molecule in a blood sample from a patient, wherein the RNA molecule comprises a nucleotide sequence according to SEQ ID NO: 1.

In a fourth aspect, the present invention relates to a method of determining the risk for a relapse of lung cancer in a patient comprising the step of:
determining the level of an RNA molecule in a blood sample from a patient, wherein the RNA molecule comprises a nucleotide sequence according to SEQ ID NO: 1.

In a fifth aspect, the present invention relates to a method of detecting a minimal residual disease in a patient having lung cancer comprising the step of:
determining the level of an RNA molecule in a blood sample from a patient, wherein the RNA molecule comprises a nucleotide sequence according to SEQ ID NO: 1.

In a sixth aspect, the present invention relates to the use of an RNA molecule for diagnosing lung cancer in a patient, monitoring the course of lung cancer in a patient, determining whether a patient responds to a therapeutic treatment of lung cancer, determining the risk for a relapse of lung cancer in a patient, and/or detecting a minimal residual disease (MRD) in a patient having lung cancer,
wherein the RNA molecule comprises a nucleotide sequence according to SEQ ID NO: 1.

In a seventh aspect, the present invention relates to the use of a kit for diagnosing lung cancer in a patient, monitoring the course of lung cancer in a patient, determining whether a patient responds to a therapeutic treatment of lung cancer, determining the risk for a relapse of lung cancer in a patient, and/or detecting a minimal residual disease in a patient having lung cancer, wherein said kit comprises:
means for determining the level of an RNA molecule in a blood sample from a patient, wherein the RNA molecule comprises a nucleotide sequence according to SEQ ID NO: 1.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

The term "comprise" or variations such as "comprises" or "comprising" according to the present invention means the inclusion of a stated integer or group of integers but not the exclusion of any other integer or group of integers. The term "consisting essentially of" according to the present invention means the inclusion of a stated integer or group of integers, while excluding modifications or other integers which would materially affect or alter the stated integer. The term "consisting of" or variations such as "consists of" according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

As used herein, the term "about" indicates a certain variation from the quantitative value it precedes. In particular, the term "about" allows a ±5% variation from the quantitative value it precedes, unless otherwise indicated or inferred. The use of the term "about" also includes the specific quantitative value itself, unless explicitly stated otherwise. For example, the expression "about 80°C" allows a variation of ±4°C, thus referring to range from 76°C to 84°C.

The similarity of nucleotide and amino acid sequences, i.e. the percentage of sequence identity, can be determined via sequence alignments. Such alignments can be carried out with several art-known algorithms, preferably with the mathematical algorithm of Karlin and Altschul (Karlin & Altschul (1993) Proc. Natl. Acad. Sci. USA 90: 5873-5877), with hmmalign (HMMER package) or with the CLUSTAL algorithm (Thompson, J. D., Higgins, D. G. & Gibson, T. J. (1994) Nucleic Acids Res. 22, 4673-80) or the CLUSTALW2 algorithm (Larkin MA, Blackshields G, Brown NP, Chenna R, McGettigan PA, McWilliam H, Valentin F, Wallace IM, Wilm A, Lopez R, Thompson JD, Gibson TJ, Higgins DG. (2007). Clustal W and Clustal X version 2.0. Bioinformatics, 23, 2947-2948).

The grade of sequence identity (sequence matching) may be calculated using e.g. BLAST, BLAT or BlastZ (or BlastX). A similar algorithm is incorporated into the BLASTN and BLASTP programs of Altschul et al. (1990) J. Mol. Biol. 215: 403-410. BLAST protein searches are performed with the BLASTP program available e.g. on the web site: http://blast.ncbi.nlm.nih.gov/Blast.cgi?PROGRAM=blastp&BLAST_PROGRAMS=blastp&P AGE_TYPE=BlastSearch&SHOW_DEFAULTS=on&LINK_LOC=blasthome.

Preferred algorithm parameters used are the default parameters as they are set on the indicated web site:
Expect threshold = 10, word size = 3, max matches in a query range = 0, matrix = BLOSUM62, gap costs = Existence: 11 Extension: 1, compositional adjustments = conditional compositional score matrix adjustment together with the database of non-redundant protein sequences (nr).

To obtain gapped alignments for comparative purposes, Gapped BLAST is utilized as described in Altschul et al. (1997) Nucleic Acids Res. 25: 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: I54-I62) or Markov random fields.

When percentages of sequence identity are referred to in the present application, these percentages are calculated in relation to the full length of the indicated reference sequence, if not specifically indicated otherwise. For example, the statement *"a nucleotide sequence having at least 80%, preferably at least 85%, more preferably at least 90%, and most preferably at least 95% or 99%, e.g. at least 80, 81, 82, 83, 84, 85, 86, 87, 88, 89, 90, 91, 92, 93, 94, 95, 96, 97, 98, or 99%, sequence identity to SEQ ID NO: XYZ"* means that the sequence identity percentage is calculated in relation to the total length of SEQ ID NO: XYZ. Herein specifically described are variants of a nucleotide sequence according to SEQ ID NO: 1 or SEQ ID NO: 2. Said variants have at least 80%, preferably at least 85%, more preferably at least 90%, and most preferably at least 95% or 99%, e.g. at least 80, 81, 82, 83, 84, 85, 86, 87, 88, 89, 90, 91, 92, 93, 94, 95, 96, 97, 98, or 99%, sequence identity to the nucleotide sequence according to SEQ ID NO: 1 or SEQ ID NO: 2.

When percentages of sequence identity between two sequences are calculated, these percentages can also be calculated in relation to the full length of the longer of the two sequences, if not specifically indicated otherwise. For example, the statement *"a nucleotide sequence having at least 80%, preferably at least 85%, more preferably at least 90%, and most preferably at least 95% or 99%, e.g. at least 80, 81, 82, 83, 84, 85, 86, 87, 88, 89, 90, 91, 92, 93, 94, 95, 96, 97, 98, or 99%, sequence identity to SEQ ID NO: XYZ"* means that the sequence identity percentage is calculated in relation to the longer sequence of the two sequences e.g. the nucleotide sequence according to SEQ ID NO: 1 in relation to the nucleotide sequence according to SEQ ID NO: 2, which is 1 nucleotide longer than the nucleotide sequence according to SEQ ID NO: 1.

The term "RNA molecule", as used herein, refers to a polymeric molecule essential in various biological roles in coding, decoding, regulation, and expression of genes. RNA and deoxyribonucleic acid (DNA) are nucleic acids. Along with lipids, proteins, and carbohydrates, nucleic acids constitute one of the four major macromolecules essential for all known forms of life. Like DNA, RNA is assembled as a chain of nucleotides, but unlike DNA, RNA is found in nature as a single strand folded onto itself, rather than a paired double strand. Cellular organisms use messenger (mRNA) to convey genetic information (using the nitrogenous bases of guanine, uracil, adenine, and cytosine, denoted by the letters G, U, A, and C) that directs synthesis of specific proteins. Some RNA molecules play an active role within cells by catalysing biological reactions, controlling gene expression or sensing and communicating responses to cellular signals. One of these active processes is protein synthesis, a universal function in which RNA molecules direct the synthesis of proteins on ribosomes. This process uses transfer RNA (tRNA) molecules to deliver amino acids to the ribosome, where ribosomal RNA (rRNA) then links amino acids together to form coded proteins.

The term "ribosomal ribonucleic acid (rRNA)", as used herein, refers to a type of noncoding RNA which is the primary component of ribosomes, essential to all cells. rRNA is a ribozyme which carries out protein synthesis in ribosomes. Ribosomal RNA is transcribed from ribosomal DNA (rDNA) and then bound to ribosomal proteins to form small and large ribosome subunits. rRNA is the physical and mechanical factor of the ribosome that forces transfer RNA (tRNA) and messenger RNA (mRNA) to process and translate the latter into proteins. Ribosomal RNA is the predominant form of RNA found in most cells; it makes up about 80% of cellular RNA despite never being translated into proteins itself. Ribosomes are composed of approximately 60% rRNA and 40% ribosomal proteins by mass.

Mammalian cells have 2 mitochondrial (12S and 16S) rRNA molecules and 4 types of cytoplasmic rRNA molecules (the 28S, 5.8S, 18S, and 5S subunits). The 28S, 5.8S, and 18S rRNAs are encoded by a single transcription unit (45S) separated by 2 internally transcribed spacers. The first spacer corresponds to the one found in bacteria and archaea, and the other spacer is an insertion into what was the 23 S rRNA in prokaryotes. The 45S rDNA is organized into 5 clusters (each has 30-40 repeats) on chromosomes 13, 14, 15, 21, and 22. These are transcribed by RNA polymerase I. The DNA for the 5S subunit occurs in tandem arrays (~200-300 true 5S genes and many dispersed pseudogenes), the largest one on the chromosome 1q41-42. 5S rRNA is transcribed by RNA polymerase III. The 18S rRNA in most eukaryotes is in the small ribosomal subunit, and the large subunit contains three rRNA species (the 5S, 5.8S, and 28S).

The present inventors identified a 28S ribosomal RNA (rRNA) fragment which is upregulated in lung cancer patients compared to healthy controls. In particular, the present inventors found that the 28S rRNA fragment is significantly upregulated in lung cancer patients compared to healthy controls. In addition, the present inventors showed that the 28S rRNA fragment is expressed more highly with increasing tumor stage in a clinical trial on lung cancer patients. After lung cancer surgery, the present inventors found that the 28S rRNA fragment level returns to normal level. Said RNA molecule can, thus, be used as biomarker for lung cancer diagnosis, monitoring, relapse control, minimal disease detection, and surgical resection status determination.

The term "28S ribosomal RNA (rRNA)", as used herein, refers to a structural ribosomal RNA (rRNA) for the large subunit (LSU) of eukaryotic cytoplasmic ribosomes, and thus, to one of the basic components of all eukaryotic cells. It has a size of 28S in mammals, hence the name.

The 28S rRNA as described herein has the following nucleotide sequence: GCCGCCGGUGAAAUACCACUAC (SEQ ID NO: 1). A variant of the 28S rRNA as described herein has the following nucleotide sequence: GCCGCCGGUGAAAUACCACUACU (SEQ ID NO: 2). The nucleotide sequence according to SEQ ID NO: 2 is, thus, 1 nucleotide longer than the nucleotide sequence according to SEQ ID NO: 1. The level of both nucleotide sequences may be determined in the methods of the present invention. By the wording, RNA molecule comprising the nucleotide sequence according to SEQ ID NO: 1 also the nucleotide sequence according to SEQ ID NO: 2 is covered.

The term "cancer", as used herein, refers to or describes a physiological condition in an individual that is typically characterized by unregulated cell growth. Examples of cancer include, but are not limited to, lung cancer, preferably non-small-cell lung carcinoma (NSCLC) or small-cell lung carcinoma (SCLS), breast cancer, cervical cancer, gastric cancer, bladder cancer, skin cancer, nasopharyngeal cancer, neuroendocrine cancer, colon cancer, urothelial cancer, liver cancer, ovarian cancer, esophageal cancer, pancreatic cancer, kidney cancer, stomach cancer, esophageal cancer, renal cancer, head and neck cancer, brain cancer, lymphatic cancer, blood cancer, squamous cell cancer, laryngeal cancer, retina cancer, prostate cancer, uterine cancer, testicular cancer, bone cancer, tonsil cancer, gullet cancer, lymphoma, and leukemia. The term "cancer", as used herein, also encompasses cancer metastases. Preferably, the cancer is selected from the group consisting of lung cancer, such as non-small-cell lung carcinoma (NSCLC) or small-cell lung carcinoma (SCLS), bladder cancer, colon cancer, gullet cancer, stomach cancer, tonsil cancer, and uterine cancer. More preferably, the cancer is lung cancer, e.g. lung cancer of stage I, II, III, or IV. Even more preferably, the lung cancer is non-small-cell lung carcinoma (NSCLC), e.g. NSCLC of stage I, II, III or IV, or small-cell lung carcinoma (SCLS), e.g. SCLS of stage I, II, III or IV. Still even more preferably, the lung cancer is non-small-cell lung carcinoma (NSCLC), e.g. NSCLC of stage I, II, III or IV.

The term "lung cancer", as used herein, refers to a disease which consists of uncontrolled cell growth in tissues of the lung. This growth may lead to metastasis, which is the invasion of adjacent tissue and infiltration beyond the lungs. The vast majority of primary lung cancers are carcinomas of the lung, derived from epithelial cells. Lung cancer is the most common cause of cancer-related death in men and women. The most common symptoms are shortness of breath, coughing (including coughing up blood), and weight loss.

The lung cancer may be lung cancer of stage I, II, III, or IV. Preferably, the lung cancer is non-small-cell lung carcinoma (NSCLC), e.g. NSCLC of stage I, II, III or IV, or small-cell lung carcinoma (SCLS), e.g. SCLS of stage I, II, III or IV. More preferably, the lung cancer is non-small-cell lung carcinoma (NSCLC), e.g. NSCLC of stage I, II, III or IV.

Lung cancer can be staged as follows:
*Stage I* means that the cancer is small. It hasn't spread to the lymph nodes or other distant organs. Stage I belongs to the early lung cancer stages. Stage I can be divided into IA and IB.
   Stage IA means the cancer is 3 cm or smaller.
   Stage IB means the cancer is between 3 cm and 4 cm. It might also be growing into structures such as: the main airway of the lung (main bronchus) or the membrane covering the lung (visceral pleura).
*Stage II* still belongs to the early lung cancer stages. Stage II can be divided into stage IIA and IIB. Part of the affected lung might have collapsed.
   Stage IIA means that the cancer is between 4 cm and 5 cm in size but there are no cancer cells in any lymph nodes.
   Stage IIB means that the cancer is up to 5 cm in size and there are cancer cells in the lymph nodes close to the affected lung. Alternatively, it is between 5 cm and 7 cm but there are no cancer cells in any lymph nodes. Alternatively, the cancer is not in any lymph nodes but has spread into one or more of the following areas: the chest wall (ribs, muscle or skin), the nerve close to the lung (the phrenic nerve), or the layers that cover the heart (mediastinal pleura and parietal pericardium). Alternatively, the cancer is less than 7 cm but there is more than one tumor in the same lobe of the lung.
*Stage III* can be divided into stage IIIA, IIIB and IIIC. It is sometimes called locally advanced lung cancer.
   In stage IIIA, the cancer is up to 5cm in size and has spread to the lymph nodes in the center of the chest on the same side as the tumor. Alternatively, the cancer it is between 5 cm and 7 cm and there is more than one tumor in the same lobe of the lung. Alternatively, the cancer has spread into one or more of the following areas just outside the lung: the chest wall (ribs, muscle or skin), the nerve close to the lung (the phrenic nerve), the layers that cover the heart (mediastinal pleura and parietal pericardium), or lymph nodes in the lung or close to the lung. Alternatively, the cancer is larger than 7 cm. It hasn't spread into lymph nodes but has spread into one or more of the following areas: the muscle under the lung (diaphragm), the center area of the chest (mediastinum), the heart, a main blood vessel, the wind pipe (trachea), the nerve that goes to the voice box (larynx), the food pipe (oesophagus), a spinal bone, or the area where the wind pipe divides (the carina). Alternatively, the cancer is in more than one lobe of the same lung and there might also be cancer cells in lymph nodes close to the affected lung.
   Stage IIIB can also mean different things. The cancer is less than 5 cm and has spread into lymph nodes in one of these places: the opposite side of the chest from the affected lung, the neck, or above the collarbone. Alternatively, the cancer is between 5 cm to 7 cm and has spread into lymph nodes in the center of the chest. Alternatively, the cancer is any size, has spread into lymph nodes in the center of the chest, and has spread into one or more of the following areas: the chest wall, the muscle under the lung (diaphragm), or the layers that cover the heart (mediastinal pleura and parietal pericardium). Alternatively, the cancer has spread into the lymph nodes in the center of the chest. The lung tumor is more than 7 cm or it has spread into a major structure in your chest such as: the heart, the wind pipe (trachea), the food pipe (oesophagus), or a main blood vessel.
   Stage IIIC means the cancer is between 5c m and 7 cm in size or has spread into one or more of the following: the nerve close to the lung (phrenic nerve) or the covering of the heart (parietal pericardium) and it has spread into lymph nodes: in the center of the chest on the opposite side from the affected lung or at the top of the lung on the same side or opposite side or above the collar bone. Alternatively, there is more than one tumor in a different lobe of the same lung. Alternatively, stage IIIC can mean the cancer is bigger than 7 cm or it has spread into one of the following: the muscle under the lung (the diaphragm), the center of the chest (mediastinum), the heart, a major blood vessel, the wind pipe (trachea), the nerve going to the voice box (the recurrent laryngeal nerve), the food pipe (oesophagus), a spinal bone, or the area where the windpipe divides (the carina) and it has spread into lymph nodes: in the center of the chest on the opposite side from the affected lung or at the top of the lung on the same side or opposite side or above the collar bone. Alternatively, there are tumors in more than one lobe of the lung.
*Stage IV* means that the lung cancer has spread. It can be designated as advanced lung cancer. It is divided into stage IVA and IVB.
   Stage IVA can mean any of the following: there is cancer in both lungs, the cancer is in the covering of the lung (the pleura) or the covering of the heart (pericardium), or there is fluid around the lungs or the heart that contains cancer cells. Alternatively, it can mean that there is a single area of cancer that has spread outside the chest to a lymph node or to an organ such as the liver or bone.
   Stage IVB means that the cancer has spread to several areas in one or more organs.

The term "(therapeutic) treatment/therapy", as used herein, relates to any treatment/therapy which improves the health status and/or prolongs (increases) the lifespan of a patient. Said treatment/therapy may eliminate the disease in a patient, arrest, inhibit, or slow the development of a disease in a patient, decrease the frequency or severity of symptoms in a patient, and/or decrease the recurrence in a patient who currently has or who previously has had a disease.

As used herein, the term "cancer therapy" refers to any protocol, method, and/or agent that can be used in the prevention, management, treatment, and/or amelioration of cancer. In particular, the term "cancer therapy", as used herein, means accomplishing one or more of the following: (i) tumor growth inhibition and/or tumor cell death, (ii) reduction of tumor marker(s), (iii) reduction of tumor lesions and metastases, (iv) reduction of tumor burden as evidenced by imaging studies (e.g. computer tomography (CT), magnetic resonance imaging (MRI), positron emission tomography (PET), etc.), and (v) reduction of tumor burden as evidenced by clinical appraisal or self-report by the patient.

Specifically, cancer therapy includes, but is not limited to, drug therapy, supportive therapy, and/or other therapy useful in the prevention, management, treatment, and/or amelioration of cancer known to one of skill in the art, such as medical personnel. More specifically, cancer therapy includes, but is not limited to, surgery, chemotherapy, targeted therapy, immunotherapy, oncolytic viral therapy, vaccination therapy, radiotherapy, laser therapy, hyperthermia therapy, and administration of a drug, or is a combination thereof, e.g. a combination of chemotherapy and immunotherapy.

The drug administered to a patient in need thereof is preferably selected from the group consisting of an immunomodulatory agent, a chemotherapeutic agent, an immunotherapeutic agent, an immunosuppressive agent, a radiotherapeutic agent, an oncolytic virus, a vaccine, and an antibody. Also, a combination of these drugs may be administered, e.g. a chemotherapeutic agent in combination with an immunotherapeutic agent. The administration of a chemotherapeutic agent in combination with an immunotherapeutic agent can be designated as immunochemotherapy.

The term "chemotherapy", as used herein, relates to a type of cancer treatment that uses one or more anti-cancer drugs, also known as chemotherapeutic agents, as part of a standardized chemotherapy regimen. Chemotherapy may be given with a curative intent (which preferably involves combinations of drugs), or it may aim to prolong life or to reduce symptoms (palliative chemotherapy). Preferably, the chemotherapy is platinum doublet chemotherapy.

The term "chemotherapeutic agent", as used herein, refers to any agent which directly or indirectly inhibits the uncontrolled growth and proliferation of cancer cells. The chemotherapeutic agent includes, but is not limited to, an alkylating agent, an antimetabolite, folinic acid, a folate antagonist, a mitotic inhibitor, an anthracycline, a topoisomerase inhibitor, an antibody, a signal transduction inhibitor, an inhibitor of angiogenesis, and/or an inhibitor of histone deacetylase. Preferably, the chemotherapeutic agent is a platinum doublet.

The term "radiation therapy", as used herein, relates to a type of cancer treatment using ionizing radiation to control or kill cancerous/malignant cells. Ionizing radiation is normally delivered by a linear accelerator. Radiation therapy may be curative in several types of cancer if they are localized to one area of the body. It may also be used as part of adjuvant therapy, to prevent tumor recurrence after surgery to remove a primary malignant tumor. Radiation therapy is synergistic with chemotherapy, and can used before, during, and after chemotherapy in susceptible cancers.

The term "immunotherapy" refers to any therapy in which one or more components of a human's or animal's immune system is (are) deliberately modulated in order to directly or indirectly achieve some therapeutic benefit, including systemic and/or local effects, and preventative and/or curative effects. Immunotherapy can involve administering one or more immunotherapeutic drugs or agents, either alone or in any combination, to a human or animal subject by any route (e.g. orally, intravenously, dermally, by injection, by inhalation, etc.), whether systemically, locally or both. Immunotherapy can involve provoking, increasing, decreasing, halting, preventing, blocking or otherwise modulating the production of cytokines, and/or activating or deactivating cytokines or immune cells, and/or modulating the levels of immune cells, and/or delivering one or more therapeutic or diagnostic substances to a particular location in the body or to a particular type of cell or tissue, and/or destroying particular cells or tissue. Immunotherapy can be used to achieve local effects, systemic effects, or a combination of both. Immunotherapy can also be used to reduce or eliminate side effects that may have been caused by other anti-cancer therapies.

Immunotherapies designed to elicit or amplify an immune response are classified as activation immunotherapies, while immunotherapies that reduce or suppress an immune response are classified as suppression immunotherapies.

An antitumor immunotherapy has broad potential and can be used to treat many different types of advanced-stage cancer owing to the durable and robust responses it elicits across a diverse spectrum of malignancies.

Immunotherapy encompasses the administration of an immunotherapeutic agent.

The term "immunotherapeutic agent", as used herein, refers to any drug, compound, or biologic that indirectly or directly restores, enhances, stimulates, or increases the body's immune response against cancer cells and/or that decreases the side effects of other anticancer therapies. Examples of common immunotherapeutic agents known in the art include, but are not limited to, checkpoint inhibitors, cytokines, cancer vaccines, antibodies such as monoclonal antibodies, and/or non-cytokine adjuvants. Alternatively, or additionally, the immunotherapeutic treatment comprises the administration of immune cells (e.g. T cells, NK, cells, dendritic cells, B cells, etc.) to the patient. Especially, said immune cells are antigen-presenting cells and/or chimeric antigen receptor T cells.

The term "immunochemotherapy", as used herein, refers to the treatment and management of a disease such as cancer by combining immunotherapy with chemotherapy. While chemotherapy uses different drugs to kill or slow the growth of cancer cells, immunotherapy uses treatments to stimulate or restore the ability of the immune system to fight cancer. Specifically, immunochemotherapy is a combinatorial anticancer therapy to improve therapeutic responses and therapeutic outcome. The immunochemotherapy combines the administration of an immunotherapeutic agent with the administration of a chemotherapeutic agent.

The term "oncolytic viral therapy", as used herein, relates to a type of cancer treatment which uses oncolytic viruses. Oncolytic viruses are viruses that kill tumor cells directly or indirectly. Oncolytic viruses have different mechanisms of action, e.g. by infecting and lysing tumor cells, generating an immune response or introducing toxins and tumor suppressor genes into tumor cells. Oncolytic viral therapy is sometimes considered as a type of immunotherapy.

The term "vaccination therapy", as used herein, relates to a type of cancer treatment which uses vaccines. Said vaccines work to to boost the body's immune system to fight cancer. Doctors give treatment vaccines to people who already have cancer. Vaccination therapy is sometimes considered as a type of immunotherapy.

The term "laser therapy", as used herein, relates to a type of cancer treatment which uses an intense, narrow beam of light to remove or destroy cancer or abnormal cells that can turn into cancer. Tumor cells absorb light of different wavelengths (or colours) than normal cells do. So, tumor cells can be targeted by selecting the proper wavelength of the laser. Laser therapy is a type of local treatment, which means it treats a specific part of the body.

The term "hyperthermia therapy", as used herein, relates to a type of cancer treatment which uses heat to treat cancer. In particular, the hyperthermia therapy is a type of treatment in which body tissue is heated to as high as 45°C to help damage and kill cancer cells with little or no harm to normal tissue. Hyperthermia to treat cancer is also called thermal therapy, thermal ablation, or thermotherapy.

The term "response", as used herein, refers to an alteration in a patient's condition that occurs as a result of or correlates with cancer treatment. In some embodiments, a response is or comprises a beneficial response. In some embodiments, a beneficial response may include stabilization of the condition (e.g. prevention or delay of deterioration expected or typically observed to occur in the absence of treatment), amelioration (e.g. reduction in frequency and/or intensity) of one or more symptoms of the condition, and/or improvement in the prospects for cure of the condition, etc. Particularly, the response is the response of the patient suffering from cancer to cancer therapy.

Specifically, a cancer patient or (control) subject suffering from cancer who has been treated with a cancer therapy is considered to "respond", have a "response", have "a positive response" or be "responsive" to the cancer therapy, if the individual shows evidence of an anti-cancer effect according to an art-accepted set of objective criteria or reasonable modification thereof, including a clinically significant benefit, such as the prevention, or reduction of severity, of symptoms, or a slowing of the progression of the cancer. It will be understood that the aforementioned terms may also be used in regard to the cancer.

By contrast, a cancer patient or (control) subject suffering from cancer who has been treated with a cancer therapy is considered "not to respond", "to lack a response", to have "a negative response" or be "non-responsive" to the cancer therapy, if the therapy provides no clinically significant benefit, such as the prevention or reduction of the severity of symptoms, or the rate of progression of cancer.

The term "diagnosing cancer", as used herein, means determining whether a patient shows signs of or suffers from cancer.

The term "monitoring the course of cancer in a patient", as used herein, means determining whether a patient has developed cancer over time or means determining the development of cancer in a patient over time, e.g. whether cancer worsens in the patient, does not worsen/is stable in the patient, or improves in the patient over time.

The term "determining whether a patient responds to a therapeutic treatment of cancer", as used herein, means evaluating whether a therapeutic approach is effective in the patient or not.

The patient may be identified as patient who responds to cancer therapy. In this case, the therapeutic treatment of cancer is continued. Specifically, the dose of the drug administered during therapeutic treatment of cancer is adjusted, e.g. decreased, and/or the numbers of administration of the drug during therapeutic treatment of cancer is adjusted, e.g. decreased. The patient may also be identified as patient who does not respond to cancer therapy. In this case, the administration schema is changed or the therapeutic treatment of cancer is changed. Specifically, the dose of the drug administered during therapeutic treatment of cancer is adjusted, e.g. increased, and/or the numbers of administration of the drug during therapeutic treatment of cancer is adjusted, e.g. increased.

Preferably, the therapeutic treatment of cancer is selected from the group consisting of surgery, chemotherapy, targeted therapy, immunotherapy, oncolytic viral therapy, vaccination therapy, radiotherapy, laser therapy, hyperthermia therapy, and administration of a drug, or is a combination thereof, e.g. chemotherapy and immunotherapy (immunochemotherapy).

More preferably, the drug is selected from the group consisting of an immunomodulatory agent, a chemotherapeutic agent, an immunotherapeutic agent, an immunosuppressive agent, a radiotherapeutic agent, an oncolytic virus, a vaccine, and an antibody.

The term "determining the risk for a relapse of cancer in a patient", as used herein, means detecting whether a patient in remission (i.e. having no symptoms or signs of disease) has a risk of getting sick again. A recurrence occurs when the cancer comes back after treatment. This can happen weeks, months, or even years after the primary or original cancer was treated. It is impossible to know for sure if the cancer will recur. The chance of recurrence depends on the type of primary cancer. Cancer recurs because small areas of cancer cells can remain in the body after treatment. Over time, these cells may multiply and grow large enough to cause symptoms or for tests to find them. When and where a cancer recurs depends on the type of cancer. Some cancers have an expected pattern of recurrence. A cancer may recur in the following ways: in the same part of the body as the primary cancer, called a local recurrence, near where the primary cancer was located, called a regional recurrence, or in another part of the body, called a distant recurrence. Recurrent cancer is named for the location where the primary cancer began, even if it recurs in another part of the body. For example, if breast cancer recurs distantly in the liver, it is still called breast cancer, not liver cancer. Doctors call it metastatic breast cancer. Metastatic means that the cancer has spread to another part of the body.

The term "minimal residual disease (MRD)", as used herein, is the name given to small numbers of cancer cells that remain in the patient during treatment, or after treatment when the patient is in remission (no symptoms or signs of disease). It is the major cause of relapse in cancer. In cancer treatment, MRD testing has several important roles: determining whether treatment has eradicated the cancer or whether traces remain, comparing the efficacy of different treatments, monitoring patient remission status as well as detecting recurrence of cancer, and choosing the treatment that will best meet those needs.

Preferably, the cancer described above is lung cancer, e.g. lung cancer of stage I, II, III, or IV. More preferably, the lung cancer is non-small-cell lung carcinoma (NSCLC), e.g. NSCLC of stage I, II, III or IV, or small-cell lung carcinoma (SCLS), e.g. SCLS of stage I, II, III or IV. Even more preferably, the lung cancer is non-small-cell lung carcinoma (NSCLC), e.g. NSCLC of stage I, II, III or IV.

The term "neoadjuvant therapy", as used herein, refers to a therapy that is administered before the planned main therapy of a tumor disease - usually surgery. Neoadjuvant therapy aims to reduce the size or extent of the cancer before using radical treatment intervention, thus, both making procedures easier and more likely to succeed and reducing the consequences of a more extensive treatment technique, which would be required if the tumor were not reduced in size or extent. Neoadjuvant therapy may, for example, comprise chemotherapy, radiotherapy, hyperthermia therapy, immunotherapy, or hormone therapy.

The term "adjuvant therapy", as used herein, refers to a therapy that is given in addition to the primary or initial therapy to maximize its effectiveness. The surgeries and complex treatment regimens used in cancer therapy have led the term to be used mainly to describe adjuvant cancer treatments. An example of such adjuvant therapy is the additional treatment usually given after surgery where all detectable disease has been removed, but where there remains a statistical risk of relapse due to the presence of undetected disease. If known disease is left behind following surgery, then further treatment is not technically adjuvant. A patient suffering, for example, from a minimal residual disease (MRD) preferably receives such an adjuvant therapy. Adjuvant therapy may, for example, comprise chemotherapy, radiotherapy, hyperthermia therapy, immunotherapy, or hormone therapy.

The term "remission", as used herein, means that a treatment reduced or eliminated the symptoms and/or signs of a disease, such as cancer. Remission may last for months, years, or the rest of life of a patient. In case of cancer, remission includes complete remission and partial remission. Cancer is considered in complete remission when there isn't any evidence of cancer on physical exam, blood work, or imaging tests. For example, if a patient has lung cancer that's in complete remission, the patient's symptoms will have improved and a computed tomography (CT) scan will show the cancer has disappeared. Complete remission doesn't mean cancer is gone forever. Cancer can come back (recur). In addition, cancer is considered in partial remission, if imaging and blood tests show cancerous tumors are at least 50% smaller than they were before treatment and/or tumor cells don't appear to be growing.

A patient is considered cured from cancer, if the patient has no signs or symptoms of cancer for at least five years after finishing treatment.

The term "recurrence", as used herein, means that symptoms and/or signs of a disease, such as cancer, came back. In other words, if a disease such as cancer is found after treatment and after a period of time when the diseases such as cancer couldn't be detected, it is called recurrence of a disease such as cancer recurrence. Local recurrence, in the case of cancer, means that the cancer is in the same place as the original cancer or very close to it. Regional recurrence, in case of cancer, means that the tumor has grown into lymph nodes or tissues near the original cancer. Distant recurrence, in case of cancer, means the cancer has spread to organs or tissues far from the original cancer.

The term "progression", as used herein, means that the symptoms and/or signs of a disease, such as cancer, become worse or increase over time. In case of cancer, the cancer (further) spreads in the body. To indicate the progression of cancer, the cancer is usually assigned to different stages with the lowest stage describing early and small cancer/tumor forms and with the higher stages categorizing the cancer that has progressed and/or spread. Most cancers are assigned one of four stages, ranging from 1 to 4.

The term "stable disease", as used herein, refers to a disease, such as cancer, that is neither decreasing nor increasing in extent or severity. In addition, the term "stable disease, as used herein, refers to a disease, such as cancer, that is neither worsening nor improving over time. In a stable patient, the patient's condition is constant. Stable" preferably means a change over time which does not exceed 10% or 20%, e.g. 1, 2, 3, 4, 5, 6, 7, 8, 9, 10, 11, 12, 13, 14, 15, 16, 17, 18, 19, or 20%. A minor change over time may be a variation which is within the accuracy of a measurement. The accuracy of a measurement depends on the measurement method used. More preferably, the level is constant over time.

The term "patient", as used herein, refers to any subject for whom it is desired to know whether she or he suffers from cancer. In particular, the term "patient", as used herein, refers to a subject suspected to be affected by cancer. The patient may be diagnosed to be affected by cancer, i.e. diseased, or may be diagnosed to be not affected by cancer, i.e. healthy.

The term "patient", as used herein, also refers to a subject which is affected by cancer, i.e. diseased. The patient may be monitored to determine whether cancer further develops in the patient over time or not. In particular, the patient may be monitored in form of a longitudinal monitoring. Thus, the course of cancer (particularly after or during treatment) may be observed in the patient. The patient may also be retested for cancer and may be diagnosed as having developed cancer over time or not.

The term "patient", as used herein, further refers to a subject suffering from cancer for whom it is desired to know whether she or he responds to cancer therapy. The patient may be identified as patient who responds to cancer therapy. In this case, the therapeutic treatment of cancer is continued. Specifically, the dose of the drug administered during therapeutic treatment of cancer is adjusted, e.g. decreased, and/or the numbers of administration of the drug during therapeutic treatment of cancer is adjusted, e.g. decreased. The patient may also be identified as patient who does not respond to cancer therapy. In this case, the administration schema is changed or the therapeutic treatment of cancer is changed. Specifically, the dose of the drug administered during therapeutic treatment of cancer is adjusted, e.g. increased, and/or the numbers of administration of the drug during therapeutic treatment of cancer is adjusted, e.g. increased.

In addition, the term "patient", as used herein, refers to any subject for whom it is desired to know whether she or he (in remission) has a risk of getting sick again or whether she or he has a risk as experiencing a relapse. The patient may be identified as patient having a risk of relapse or not.

Moreover, the term "patient", as used herein, refers to any subject for whom it is desired to know whether the cancer treatment has eradicated the cancer or whether traces remain. The patient (in remission) may be identified as patient who still carries a small numbers of cancer cells after treatment or not. Such a patient suffers from a minimal residual disease (MRD). A patient suffering from a minimal residual disease (MRD) is preferably assigned to/eligible for adjuvant therapy.

It should be noted that a patient that is diagnosed as being healthy, i.e. not suffering from cancer, may possibly suffer from another disease or condition not tested/known.

The patient may further be a cancer patient who is in remission, in which the cancer is stable or in which the cancer has progressed.

The patient may be any mammal, including both a human and another mammal, e.g. an animal such as a rabbit, mouse, rat, or monkey. Human individuals are particularly preferred.

The term "(control) subject", as used herein, refers to a subject known to be not affected by cancer (negative control), i.e. healthy.

The term "(control) subject", as used herein, also refers to a subject known to be affected by cancer (positive control), i.e. diseased. Said (control) subject may have developed an advanced form of cancer, e.g. cancer of a higher grade or stage.

The term "(control) subject", as used herein, further refers to a subject known be a responder of a therapeutic treatment of cancer or known to be a non-responder of a therapeutic treatment of cancer.

In addition, the term "(control) subject", as used herein, further refers to a successfully treated cancer patient.

It should be noted that a (control) subject which is known to be healthy, i.e. not suffering from cancer, may possibly suffer from another disease or condition not tested/known.

The (control) subject may be any mammal, including both a human and another mammal, e.g. an animal such as a rabbit, mouse, rat, or monkey. Human healthy individuals are particularly preferred.

The term "blood sample", as used herein, encompasses whole blood or a blood fraction. Preferably, the blood fraction is selected from the group consisting of a blood cell fraction, plasma, and serum. In particular the blood fraction is selected from the group consisting of a blood cell fraction and plasma or serum. For example, the blood cell fraction encompasses erythrocytes, leukocytes, and/or thrombocytes. More preferably, the blood cell fraction is a fraction of leukocytes or a mixture of erythrocytes, leukocytes, and thrombocytes.

It is preferred that the blood sample has a volume of between 0.01 and 20 ml, more preferably of between 0.1 and 10 ml, even more preferably of between 0.5 and 8 ml and most preferably of between 1 and 5 ml.

Said blood sample may be provided by removing blood from a patient or (control) subject, but may also be provided by using a previously isolated sample. For example, a blood sample may be taken from a patient or (control) subject by conventional blood collection techniques.

The blood sample may further be obtained from a patient or (control) subject prior to the initiation of a therapeutic treatment, during the therapeutic treatment, and/or after the therapeutic treatment. If the blood sample is obtained from at least one (control) subject, e.g. from at least 1, 2, 3, 4, 5, 6, 7, 8, 9, 10, 20, 30, 40, 50, 60, 70, 80, 90, 100, 150, 200, 250, 300, 400, 500, or 1,000 (control) subject(s), it is designated as "reference blood sample". Preferably, the reference blood sample is from the same source than the blood sample of the patient to be tested, e.g. both are whole blood samples or blood cell fractions. It is further preferred that both are from the same species, e.g. from a human. It is also (alternatively or additionally) preferred that the measurements of the reference blood sample of the (control) subject and the blood sample of the patient to be tested are identical, e.g. both have an identical volume. It is particularly preferred that the reference blood sample and the blood sample are from (control) subjects/patients of the same sex and similar age.

The whole blood sample may be collected by means of a blood collection tube. It is, for example, collected in a PAXgene Blood RNA tube, in a Tempus Blood RNA tube, in an EDTA-tube, in a Na-citrate tube, Heparin-tube, or in an ACD-tube (Acid citrate dextrose). Alternatively, the whole blood sample may be collected in a blood collection tube containing cell-free nucleic acid stabilizing chemical agents, such as glutaraldehyde, formaldehyde, or similar (e.g. Streck cfRNA BCT tube, Streck cfDNA BCT tube), and others, or cellular crowding agents, such as polyethyleneglycol (PEG) (e.g. Norgen cfDNA/cfRNA preservation tube), and others.

The blood sample, in particular whole blood sample, as used herein, may also be collected by means of a bloodspot technique, e.g. using a Mitra Microsampling Device. This technique requires smaller sample volumes, typically 45-60 µl for humans or less. For example, the whole blood may be extracted from the patient via a finger prick with a needle or lancet. Thus, the whole blood sample may have the form of a blood drop. Said blood drop is then placed on an absorbent probe, e.g. a hydrophilic polymeric material such as cellulose, which is capable of absorbing the whole blood. Once sampling is complete, the blood spot is dried in air before transferring or mailing to labs for processing. Because the blood is dried, it is not considered hazardous. Thus, no special precautions need be taken in handling or shipping. Once at the analysis site, the desired components, e.g. the RNA molecule as described herein, are extracted from the dried blood spots into a supernatant which is then further analyzed. In this way, the level of the RNA molecule is determined. This technique is suitable for monitoring patients having cancer at home (on a home care/home sampling basis) or for screening purposes.

In the methods described herein, the level of an RNA molecule is determined in a blood sample of a patient. The term "level", as used herein, refers to an amount (measured for example in grams, mole, or ion counts) or concentration (e.g. absolute or relative concentration, e.g. reads per million (RPM) or NGS counts) of the RNA molecule. The term "level", as used herein, also comprises scaled, normalized, or scaled and normalized amounts or values (e.g. RPM). In particular, the level of the RNA molecule is determined by sequencing, preferably next generation sequencing (e.g. ABI SOLID, Illumina Genome Analyzer, Roche 454 GS FL, BGISEQ), nucleic acid hybridization (e.g. microarray or beads), nucleic acid amplification (e.g. PCR, RT-PCR, qRT-PCR, or high-throughput RT-PCR), polymerase extension, mass spectrometry, flow cytometry (e.g. LUMINEX), or any combination thereof. Specifically, the level of the RNA molecule is the expression level of said RNA molecule.

Those of skill in the art will appreciate that, in many embodiments described herein, the determined RNA molecule level is compared with an appropriate RNA molecule "reference level". Specifically, the level of the RNA molecule is compared to a reference level of said RNA molecule. More specifically, the reference level of a RNA molecule is determined in a blood sample of (control) subjects. Even more specifically, the reference level is determined empirically by measuring a number of reference blood samples from subjects suffering from cancer known to be responders or non-responders to cancer therapy or by measuring a number of reference blood samples from subjects suffering from cancer having a known good or poor survival prognosis. Typically, as would be understood by those skilled in the art, the reference level is determined under conditions comparable to those utilized to determine or analyze the RNA molecule level in a blood sample of a patient.

The reference level may also be a cut-off or threshold level. Typically, a cut-off or threshold level can be determined experimentally, empirically, or theoretically. A cut-off or threshold level can also be arbitrarily selected based upon the existing experimental and/or clinical conditions, as would be recognized by a person of ordinary skilled in the art. The cut-off or threshold level must be determined in order to obtain the optimal sensitivity and specificity according to the function of the test and the benefit/risk balance (clinical consequences of false positive and false negative). Typically, the optimal sensitivity and specificity (and so the threshold level) can be determined using a Receiver Operating Characteristic (ROC) curve based on experimental data. For example, after determining the RNA molecule level in a group of a reference, one can use algorithmic analysis for the statistic treatment of the measured RNA molecule level in samples to be tested, and thus obtain a classification standard having significance for sample classification. The full name of ROC curve is receiver operator characteristic curve, which is also known as receiver operation characteristic curve. It is mainly used for clinical biomarker tests. The ROC curve is a comprehensive indicator that reflects the continuous variables of true positive rate (sensitivity) and false positive rate (1 -specificity). It reveals the relationship between sensitivity and specificity with the image composition method. A series of different cut-off or threshold levels are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of a prediction/prognosis/diagnosis. On the ROC curve, the point closest to the far upper left of the coordinate diagram is a critical point having both high sensitivity and high specificity values. The AUC value of the ROC curve is between 1.0 and 0.5. When AUC > 0.5, the predicted/prognosed/diagnosed result gets better and better as AUC approaches 1.

The term "classifier", as used herein, refers to a prediction/prognostic model which allows to distinguish between or characterize samples by classifying a given sample into a predetermined class based on certain characteristics of said sample. For example, a classification is capable of predicting with a relatively high sensitivity and specificity if a blood sample of a patient of unknown response prediction belongs to the class of one of two given classes; each class representing a predicted estimated response. The output may be given as a probability of belonging to either class of between 0-1. A classification is also capable of predicting with a relatively high sensitivity and specificity if a blood sample of a patient of unknown survival prognosis belongs to the class of one of two given classes; each class representing a predicted estimated survival. The output may be given as a probability of belonging to either class of between 0-1. Specifically, a classifier allows to distinguish responders to cancer therapy from non-responders to cancer therapy.

In the context of the present invention, the term "kit of parts (in short: kit)" is understood to be any combination of at least some of the components identified herein, which are combined, coexisting spatially, to a functional unit, and which can contain further components.

### Embodiments of the invention

The present invention will now be further described. In the following passages, different aspects of the invention are defined in more detail.

The present inventors identified a 28S ribosomal RNA (rRNA) fragment which is upregulated between lung cancer patients and healthy controls. In particular, the present inventors found that the 28S rRNA fragment is significantly upregulated in lung cancer patients compared to healthy controls. In addition, the present inventors showed that the 28S rRNA fragment is expressed more highly with increasing tumor stage in a clinical trial on lung cancer patients. After lung cancer surgery, the present inventors found that the 28S rRNA fragment level returns to normal level. Said RNA molecule can, thus, be used as biomarker for lung cancer diagnosis, monitoring, relapse control, minimal disease detection, and surgical resection status determination.

Thus, in a first aspect, the present invention relates to a (an) (*in vitro*) method of diagnosing lung cancer in a patient (suspected of suffering from lung cancer) comprising the step of:
determining the level of an RNA molecule in a blood sample from a patient (suspected of suffering from lung cancer), wherein the RNA molecule comprises a nucleotide sequence according to SEQ ID NO: 1.

In one embodiment, the level of the RNA molecule is compared to a reference level of said RNA molecule. Thus, in one particular embodiment, the present invention relates to a (an *in vitro*) method of diagnosing lung cancer in a patient comprising the steps of:
(i) determining the level of an RNA molecule in a blood sample from a patient, and
(ii) comparing the level of the RNA molecule to a reference level of said RNA molecule, wherein the RNA molecule comprises a nucleotide sequence according to SEQ ID NO: 1.

The above comparison allows to diagnose lung cancer in a patient, in particular in a patient suspected of suffering from lung cancer. The patient may be diagnosed as suffering from lung cancer, i.e. being diseased, or as not suffering from lung cancer, i.e. being healthy.

The reference level may be any level which allows to determine whether the patient suffers from lung cancer or not. It may be obtained from (control) subjects (i.e. subjects different from the patient to be tested such as healthy subjects and/or subjects known to have lung cancer) or from the same patient. In the latter case, the patient may be retested for lung cancer, e.g. in the form of a longitudinal monitoring. It may be determined that the patient is now affected by lung cancer or still not affected by lung cancer.

In particular, the reference level is the level of the RNA molecule determined empirically by measuring a number of reference blood samples from
healthy subjects, and/or
subjects having lung cancer/known to have lung cancer.

For example,
the reference level is determined from at least 2, at least 10, at least 50, at least 100, at least 200, at least 300, at least 400, at least 500, at least 1000, at least 1500, at least 2000 at least 5000 reference samples from healthy subjects, and/or
the reference level is determined from at least 2, at least 10, at least 50, at least 100, at least 200, at least 300, at least 400, at least 500, at least 1000, at least 1500, at least 2000 at least 5000 reference samples from subjects having lung cancer/known to have lung cancer.

Specifically, the reference level is an average reference level. It is determined by measuring reference levels of control subjects (e.g. healthy subjects or subjects having lung cancer/known to have lung cancer) and calculating the "average" value (e.g. mean, median or modal value) thereof. It is preferred that the reference blood sample is from the same source (e.g. blood cells, serum, or plasma) than the blood sample isolated from the patient to be tested. It is further preferred that the reference level is obtained from control subjects (e.g. healthy subjects or subjects having lung cancer/known to have lung cancer) of the same gender (e.g. female or male) and/or of a similar age/phase of life (e.g. adults or elderly) than the patient to be tested. Particularly, the reference level represents an average value of the RNA molecule in a healthy population and/or the reference level represents an average value of the RNA molecule in a population of subjects having lung cancer/known to have lung cancer. More particularly, the population group of subjects having lung cancer/known to have lung cancer includes subjects of all lung cancer stages, e.g. I, II, III, and IV, (mixed population, specifically equally represented).

In one preferred embodiment
the reference level is the level of the RNA molecule determined empirically by measuring a number of reference blood samples from healthy subjects, and wherein the level of the RNA molecule above the reference level indicates that the patient suffers from lung cancer, and/or
the reference level is the level of the RNA molecule determined empirically by measuring a number of reference blood samples from subjects having lung cancer/known to have lung cancer, and wherein the level of the RNA molecule comparable with the reference level indicates that the patient suffers from lung cancer.

Preferably, the level of the RNA molecule is at least 0.6-fold or 0.7-fold, more preferably at least 0.8-fold or 0.9-fold, even more preferably at least 1.2-fold or 1.5-fold, and still even more preferably at least 2.0-fold or 3.0-fold above the reference level. For example, the level of the RNA molecule is at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2. 1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold above the reference level.

"Comparable" in the above context preferably means that the level varies over time between 0 and < 20%, specifically between 0 and < 10%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Comparable" in this respect may also mean that the detected level variation is within the accuracy of a measurement. The accuracy of a measurement depends on the measurement method used. The level may be constant over time.

Alternatively, the level of the RNA molecule is compared to a threshold value indicative for lung cancer and wherein the level of the RNA molecule above this threshold value indicates that the patient has lung cancer.

Preferably, the lung cancer is non-small-cell lung carcinoma (NSCLC) or small-cell lung carcinoma (SCLS). More preferably, the lung cancer is non-small-cell lung carcinoma (NSCLC).

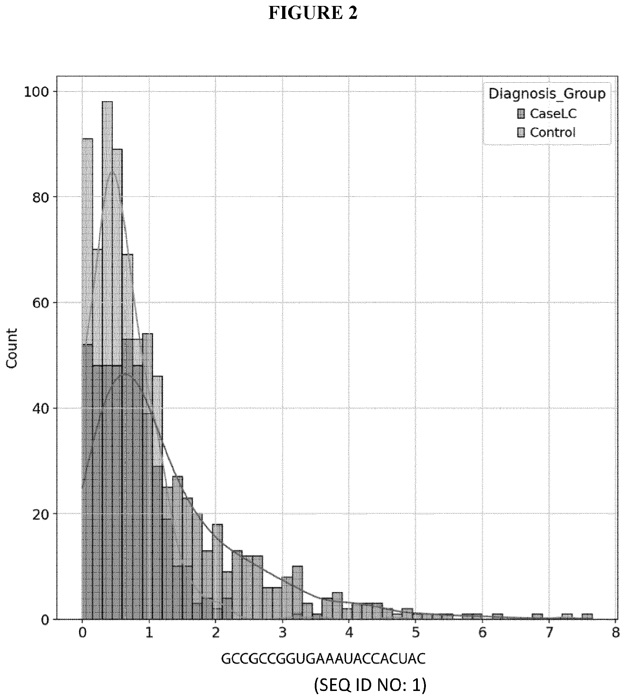

**FIGURE 2** clearly shows that the above-mentioned RNA molecule is increased in patients suffering from lung cancer compared to healthy controls.

In a second aspect, the present invention relates to a (an *in vitro*) method of monitoring the course of lung cancer in a patient (suffering from lung cancer or being healthy) comprising the step of:
determining the level of an RNA molecule in a blood sample from a patient (suffering from lung cancer or being healthy), wherein the RNA molecule comprises a nucleotide sequence according to SEQ ID NO: 1.

In one embodiment, the level of the RNA molecule is compared to a reference level of said RNA molecule. Thus, in one particular embodiment, the present invention relates to a (an *in vitro*) method of monitoring the course of lung cancer in a patient comprising the steps of:
(i) determining the level of an RNA molecule in a blood sample from a patient, and
(ii) comparing the level of the RNA molecule to a reference level of said RNA molecule, wherein the RNA molecule comprises a nucleotide sequence according to SEQ ID NO: 1.

The above comparison allows to monitor/determine the course of lung cancer in a patient suffering from lung cancer. It may be determined that lung cancer worsens in the patient, that lung cancer does not worsen/is stable in the patient, or that lung cancer improves in the patient. The above comparison also allows to monitor/determine whether lung cancer has developed in a patient.

The reference level may be any level which allows to monitor or detect lung cancer in a patient. It may be obtained from (control) subjects (i.e. subjects different from the patient to be tested such as subjects having lung cancer/known to have lung cancer or healthy subjects).

In particular, the reference level is the level of the RNA molecule determined empirically by measuring a number of reference blood samples from
healthy subjects, and/or
subjects having lung cancer/known to have lung cancer, e.g. subjects having lung cancer/known to have lung cancer of stage I, II, III, or IV. Stages I and II are early lung cancer stages. Stage III is a locally advanced lung cancer stage. Stage IV is an advanced lung cancer stage.

For example,
the reference level is determined from at least 2, at least 10, at least 50, at least 100, at least 200, at least 300, at least 400, at least 500, at least 1000, at least 1500, at least 2000 at least 5000 reference samples from healthy subjects, and/or
the reference level is determined from at least 2, at least 10, at least 50, at least 100, at least 200, at least 300, at least 400, at least 500, at least 1000, at least 1500, at least 2000 at least 5000 reference samples from subjects having lung cancer/known to have lung cancer, e.g. subjects having lung cancer/known to have lung cancer of stage I, II, III, or IV.

Specifically, the reference level is an average reference level. It is determined by measuring reference levels of control subjects (e.g. healthy subjects or subjects having lung cancer/known to have lung cancer) and calculating the "average" value (e.g. mean, median or modal value) thereof. It is preferred that the reference blood sample is from the same source (e.g. blood cells, serum, or plasma) than the blood sample isolated from the patient to be tested. It is further preferred that the reference level is obtained from control subjects (e.g. healthy subjects or subjects having lung cancer/known to have lung cancer) of the same gender (e.g. female or male) and/or of a similar age/phase of life (e.g. adults or elderly) than the patient to be tested. Particularly, the reference level represents an average value of the RNA molecule in a healthy population and/or the reference level represents an average value of the RNA molecule in a population of subjects having lung cancer/known to have lung cancer. More particularly, the population group of subjects having lung cancer/known to have lung cancer includes subjects of a specific lung cancer stage, e.g. I, II, III or IV, or subjects of all lung cancer stages, e.g. I, II, III, and IV (mixed population, specifically equally represented).

In one preferred embodiment
(i) the reference level is the level of the RNA molecule determined empirically by measuring a number of reference blood samples from healthy subjects, and wherein the level of the RNA molecule above the reference level indicates that the patient has developed lung cancer,
(ii) the reference level is the level of the RNA molecule determined empirically by measuring a number of reference blood samples from subjects having lung cancer/known to have lung cancer of a specific stage, e.g. lung cancer of stage I, II, III, or IV, whereby the stage is comparable with or higher as the stage of lung cancer of the patient to be tested, and wherein the level of the RNA molecule above the reference level indicates that the lung cancer worsens in the patient,
(iii) the reference level is the level of the RNA molecule determined empirically by measuring a number of reference blood samples from subjects having lung cancer/known to have lung cancer of a specific stage, e.g. lung cancer of stage I, II, III, or IV, whereby the stage is comparable with or lower as the stage of lung cancer of the patient to be tested, and wherein the level of the RNA molecule below the reference level indicates that the lung cancer improves in the patient, or
(iv) the reference level is the level of the RNA molecule determined empirically by measuring a number of reference blood samples from subjects having lung cancer/known to have lung cancer of a specific stage, e.g. lung cancer of stage I, II, III, or IV, whereby the stage is the same stage as the stage of lung cancer of the patient to be tested, and wherein the level of the RNA molecule comparable with the reference level indicates that the patient is stable or that lung cancer is not progressing in the patient.

Preferably, the level of the RNA molecule is at least 0.6-fold or 0.7-fold, more preferably at least 0.8-fold or 0.9-fold, even more preferably at least 1.2-fold or 1.5-fold, and still even more preferably at least 2.0-fold or 3.0-fold above/below the reference level. For example, the level of the RNA molecule is at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2. 1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold above/below the reference level.

"Comparable" in the above context preferably means that the level varies over time between 0 and < 20%, specifically between 0 and < 10%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Comparable" in this respect may also mean that the detected level variation is within the accuracy of a measurement. The accuracy of a measurement depends on the measurement method used. Preferably, the level is constant over time.

As already mentioned above, the reference level may be any level which allows to monitor or detect lung cancer in a patient. It may be obtained from (control) subjects (i.e. subjects different from the patient to be tested such as subjects known to have lung cancer or healthy subjects). It may also be obtained from the same individual.

Accordingly, in one alternative or additional embodiment, said monitoring comprises determining the level of the RNA molecule in a blood sample obtained from a patient at a first point in time and in at least one further blood sample obtained from the (same) patient at a later point in time and comparing said levels determined at the different time points. Thus, in one particular embodiment, the present invention relates to a (an *in vitro)* method of monitoring the course of lung cancer in a patient comprising the steps of:
(i) determining the level of the RNA molecule in a blood sample obtained from a patient at a first point in time and in at least one further blood sample obtained from the (same) patient at a later point in time, and
(ii) comparing said levels determined at the different time points,
wherein the RNA molecule comprises a nucleotide sequence according to SEQ ID NO: 1.

This proceeding allows to monitor lung cancer in a patient (being healthy or suffering from lung cancer) over an extended period of time, such as months or years, e.g. 1, 2, 3, 4, 5, 6, 7, 8, 9, 10, or 11 month(s), 1, 2, 3, 4, or 5 year(s).

In one preferred embodiment, the level of the RNA molecule which
(i) increases over time indicates that the patient has developed lung cancer or that lung cancer is worsening in the patient,
(ii) does not change over time indicates that the patient is stable or that lung cancer is not progressing in the patient, or
(iii) decreases over time indicates that lung cancer is improving in the patient. In one more preferred embodiment,

(i) the patient was healthy at the first point in time and the level which increases over time indicates that the patient has developed lung cancer,
(ii) the patient had lung cancer at the first point in time and the level which increases over time indicates that lung cancer is worsening in the patient,
(iii) the patient had lung cancer at the first point in time and the level which does not (significantly) change over time indicates that lung cancer is not progressing in the patient, or
(iv) the patient had lung cancer at the first point in time and the level which decreases over time indicates that lung cancer is improving in the patient.

Preferably, the increase is at least 0.6-fold or 0.7-fold, more preferably at least 0.8-fold or 0.9-fold, even more preferably at least 1.2-fold or 1.5-fold, and still even more preferably at least 2.0-fold or 3.0-fold over time.

For example, the increase may be at least 0.6-fold, at least 0.7-fold, at least 0.8-fold, at least 0.9-fold, at least 1.0-fold, at least 1. 1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold over time.

Preferably, the decrease is at least 0.6-fold or 0.7-fold, more preferably at least 0.8-fold or 0.9-fold, even more preferably at least 1.2-fold or 1.5-fold, and still even more preferably at least 2.0-fold or 3.0-fold over time.

For example, the decrease may be at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold over time.

A "significant change" in the above context preferably means a change over time which exceeds 10% or 20%, e.g. 10, 20, 30, 40, or 50%. A minor change over time may be a variation which is within the accuracy of a measurement. The accuracy of a measurement depends on the measurement method used. The level may be constant over time.

"Stable" in the above context preferably means a change over time which does not exceed 10% or 20%, e.g. 1, 2, 3, 4, 5, 6, 7, 8, 9, 10, 11, 12, 13, 14, 15, 16, 17, 18, 19, or 20%. A minor change over time may be a variation which is within the accuracy of a measurement. The accuracy of a measurement depends on the measurement method used. Preferably, the level is constant over time.

The time period between the first point in time and the later point(s) in time preferably amounts to at least 1 day, at least 2 days, at least 3 days, at least 4 days, at least 5 days, at least 6 days, at least 7 days (1 week), at least 2 weeks, at least 3 weeks, at least 4 weeks, at least 1 month, at least 2 months, at least 3 months, at least 4 months, at least 5 months, at least 6 months, at least 7 months, at least 8 months, at least 9 months, at least 10 months, at least 11 months, at least 12 months (1 year), at least 24 months (2 years), at least 3 years, at least 4 years, at least 5 years, at least 6 years, at least 7 years, at least 8 years, at least 9 years, or at least 10 years. For example, the individual may be routinely checked, e.g. once or twice a year. The patient may be (re)tested at 2, 3, 4, 5, 6 7, 8, 9, or 10 time points (first point in time and further point(s) in time).

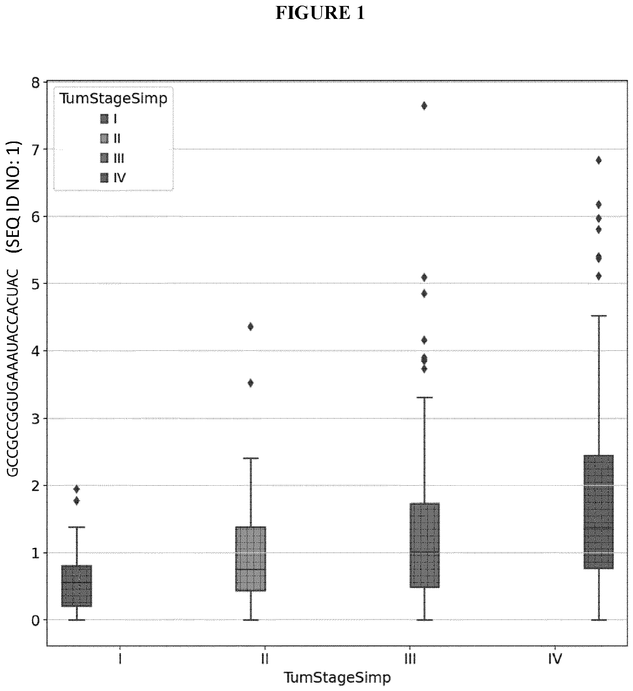

**FIGURE 1** clearly shows that the above-mentioned RNA molecule is expressed more highly with increasing tumor stage in a clinical trial on lung cancer patients.

In addition to the monitoring of lung cancer in a patient, the therapeutic treatment of lung cancer can be monitored. In particular, the patient has received or had received a therapeutic treatment of lung cancer.

Preferably, the therapeutic treatment of lung cancer is selected from the group consisting of surgery, chemotherapy, targeted therapy, immunotherapy, oncolytic viral therapy, vaccination therapy, radiotherapy, laser therapy, hyperthermia therapy, and administration of a drug, or is a combination thereof, e.g. chemotherapy and immunotherapy (immunochemotherapy). More preferably, the drug is selected from the group consisting of an immunomodulatory agent, a chemotherapeutic agent, an immunotherapeutic agent, an immunosuppressive agent, a radiotherapeutic agent, an oncolytic virus, a vaccine, and an antibody. The drug may also be a drug combination, e.g. a combination of a chemotherapeutic agent and an immunotherapeutic agent. The administration of a chemotherapeutic agent in combination with an immunotherapeutic agent can be designated as immunochemotherapy.

The patient may receive a therapeutic treatment during the complete monitoring process (e.g. the administration of a drug) or may receive a therapeutic treatment before, at, or after a first point in time (e.g. the administration of a drug) and may be retested at a later point in time. In particular, said first point in time may be before the initiation of a therapeutic treatment and said later point in time may be during the therapeutic treatment and/or after the therapeutic treatment. If the therapeutic treatment encompasses the administration of a drug and the patient responds to said treatment, the drug administration may be continued, the dose of the drug may be reduced, or the drug administration may be stopped. If the therapeutic treatment encompasses the administration of a drug and the patient does not respond to said treatment, the dose of the drug may be increased, the drug may be changed, or the therapy mode may be changed, e.g. from drug administration to surgery, laser therapy, or hyperthermia therapy.

Due to the therapeutic treatment, an increased level of the RNA molecule can be decreased. In this way, the (overall) condition of the patient having lung cancer can be improved.

In one more preferred embodiment,
(i) the patient has received or had received a therapeutic treatment for lung cancer and the level which decreases over time indicates that the patient responds to said treatment, or
(ii) the patient has received or had received a therapeutic treatment for lung cancer and the level which is stable or increases over time indicates that the patient does not respond to said treatment.

Preferably, the increase is at least 0.6-fold or 0.7-fold, more preferably at least 0.8-fold or 0.9-fold, even more preferably at least 1.2-fold or 1.5-fold, and still even more preferably at least 2.0-fold or 3.0-fold over time.

For example, the increase may be at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold over time.

Preferably, the decrease is at least 0.6-fold or 0.7-fold, more preferably at least 0.8-fold or 0.9-fold, even more preferably at least 1.2-fold or 1.5-fold, and still even more preferably at least 2.0-fold or 3.0-fold over time.

For example, the decrease may be at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold over time.

"Stable" in the above context preferably means a change over time which does not exceed 10% or 20%, e.g. 1, 2, 3, 4, 5, 6, 7, 8, 9, 10, 11, 12, 13, 14, 15, 16, 17, 18, 19, or 20%. A minor change over time may be a variation which is within the accuracy of a measurement. The accuracy of a measurement depends on the measurement method used. Preferably, the level is constant over time.

Preferably, the lung cancer is non-small-cell lung carcinoma (NSCLC) or small-cell lung carcinoma (SCLS). More preferably, the lung cancer is non-small-cell lung carcinoma (NSCLC).

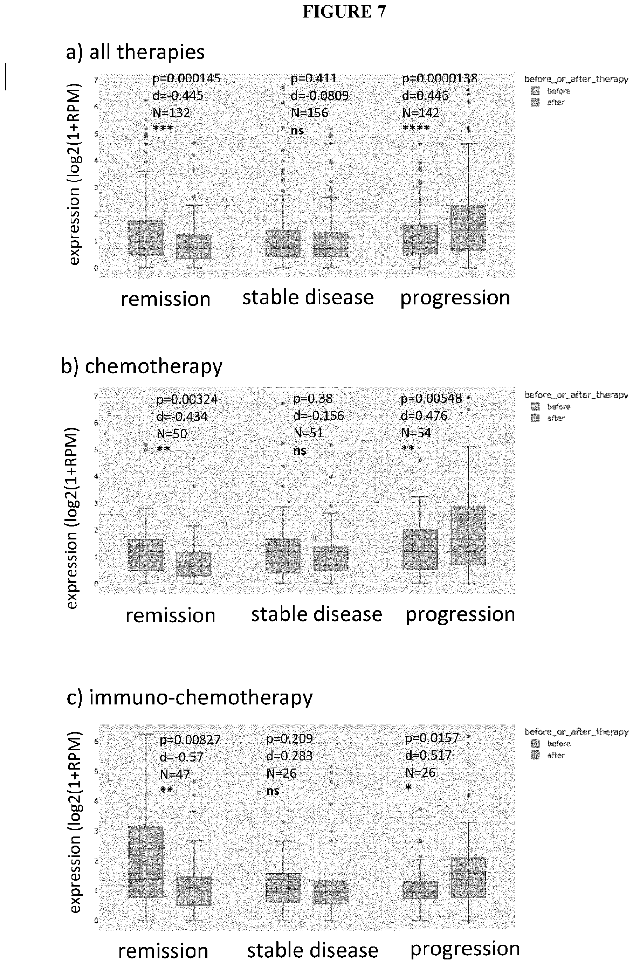

The data in **FIGURE 7** clearly show that the above-mentioned RNA molecule allows to monitor the course of lung cancer in a patient, specifically under therapeutic treatment of cancer/anti-cancer therapy such as chemotherapy or immunochemotherapy. In **FIGURE 7****,** the distribution of the above-mentioned RNA molecule in lung cancer samples before and after anti-cancer therapy of a patient is shown. The clinical assessments by the oncologist at the time of the follow-up visit (after therapy) summarize the development of the disease (since the previous visit) as either remission (disease improving), stable disease (no change), and progression (disease worsening). The level of the above-mentioned RNA molecule mirrors this assessment by (i) significantly decreasing during remission, which shows that the lung cancer is improving in the patient, (ii) significantly increasing during progression, which shows that the lung cancer is worsening in the patient, and (iii) remaining constant during stable disease periods (no significant difference), which shows that the lung cancer is not progressing in the patient.

In a third aspect, the present invention relates to a (an *in vitro*) method of determining whether a patient responds to a therapeutic treatment of lung cancer comprising the step of: determining the level of an RNA molecule in a blood sample from a patient, wherein the RNA molecule comprises a nucleotide sequence according to SEQ ID NO: 1.

It is preferred that the patient is a patient to whom at least once (1, 2, or 3 times)
a drug to be used in said therapeutic treatment, and/or
another form of therapeutic treatment
has been administered or had been administered.

The drug can also be a drug combination of at least two different medicaments, e.g. a combination of a chemotherapeutic agent and an immunotherapeutic agent. The administration of a chemotherapeutic agent in combination with an immunotherapeutic agent can be designated as immunochemotherapy.

The way of administration may be oral, nasal, rectal, parenteral, or topical. Parental administration includes subcutaneous, intracutaneous, intramuscular, intravenous or intraperitoneal administration.

It is further preferred that the blood sample is isolated from the patient after at least the first (e.g. first, second, or third) administration of said drug and/or after at least the first (e.g. first, second, or third) administration of another form of therapeutic treatment (than the administration of a drug). It is particularly preferred that the blood sample is isolated from the patient in a time period of between 2 weeks and 1 day after at least the first (e.g. first, second, or third) administration of said drug and/or after at least the first (e.g. first, second, or third) administration of another form of therapeutic treatment (than the administration of a drug). It is particularly more preferred that the blood sample is isolated from the patient in a time period of between 1 weeks and 1 day after at least the first (e.g. first, second, or third) administration of said drug and/or after at least the first (e.g. first, second, or third) administration of another form of therapeutic treatment (than the administration of a drug). For example, the blood sample is isolated from the patient 1, 2, 3, 4, 5, 6, day(s), 1, 2, 3 week(s) after at least the first (e.g. first, second, or third) administration of said drug and/or after at least the first (e.g. first, second, or third) administration of another form of therapeutic treatment (than the administration of a drug).

In one embodiment, the level of the RNA molecule is compared to a reference level of said RNA molecule. Thus, in one particular embodiment, the present invention relates to a (an *in vitro*) method of determining whether a patient responds to a therapeutic treatment of lung cancer comprising the steps of:
(i) determining the level of an RNA molecule in a blood sample from a patient, and
(ii) comparing the level of the RNA molecule to a reference level of said RNA molecule,
wherein the RNA molecule comprises a nucleotide sequence according to SEQ ID NO: 1, is a fragment thereof, or has a sequence having at least 80%, preferably at least 85%, more preferably at least 90%, and most preferably at least 95% or 99%, e.g. at least 80, 81, 82, 83, 84, 85, 86, 87, 88, 89, 90, 91, 92, 93, 94, 95, 96, 97, 98, or 99%, sequence identity thereto.

The above comparison allows to evaluate whether a therapeutic approach is effective in the patient or not.

The reference level may be any level which allows to determine whether a therapeutic treatment is effective in the patient or not. It may be obtained from (control) subjects (i.e. subjects different from the patient to be tested such as subjects having lung cancer/known to have lung cancer) or from the same individual.

In one preferred embodiment, the reference level is the level of the RNA molecule determined empirically by measuring a number of reference blood samples from subjects having lung cancer/known to have lung cancer.

For example, the reference level is determined from at least 2, at least 10, at least 50, at least 100, at least 200, at least 300, at least 400, at least 500, at least 1000, at least 1500, at least 2000 at least 5000 reference samples from subjects having lung cancer/known to have lung cancer. Specifically, the reference level is an average reference level. It is determined by measuring reference levels of subjects having lung cancer/known to have lung cancer and calculating the "average" value (e.g. mean, median or modal value) thereof. It is preferred that the reference blood sample is from the same source (e.g. blood cells, serum, or plasma) than the blood sample isolated from the patient to be tested. It is further preferred that the reference level is obtained from subjects having lung cancer/known to have lung cancer of the same gender (e.g. female or male) and/or of a similar age/phase of life (e.g. adults or elderly) than the patient to be tested. Particularly, the reference level represents an average value of the RNA molecule in a population of subjects having lung cancer/known to have lung cancer. More particularly, this population group includes subjects of a specific lung cancer stage, e.g. I, II, III or IV, or subjects of all lung cancer stages, e.g. I, II, III, and IV (mixed population, specifically equally represented).

In one more preferred embodiment,
the level of the RNA molecule below the reference level indicates that the patient responds to said treatment of lung cancer,
the level of the RNA molecule comparable with the reference level indicates that the patient does not respond to said treatment of lung cancer, or
the level of the RNA molecule above the reference level indicates that the patient does not respond to said treatment of lung cancer.

Preferably, the level of the RNA molecule is at least 0.6-fold or 0.7-fold, more preferably at least 0.8-fold or 0.9-fold, even more preferably at least 1.2-fold or 1.5-fold, and still even more preferably at least 2.0-fold or 3.0-fold above/below the reference level. For example, the level of the RNA molecule is at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2. 1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold above/below the reference level.

"Comparable" in the above context preferably means that the level varies between 0 and < 20%, specifically between 0 and < 10%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Comparable" in this respect may also mean that the detected level variation is within the accuracy of a measurement. The accuracy of a measurement depends on the measurement method used. Preferably, the level is constant over time.

In one alternative or additionally preferred embodiment,
the reference level is the level determined in a reference blood sample isolated from the (same) patient prior to the administration of
said drug, and/or
another form of therapeutic treatment.

The drug can also be a drug combination of at least two different medicaments, e.g. a combination of a chemotherapeutic agent and an immunotherapeutic agent. The administration of a chemotherapeutic agent in combination with an immunotherapeutic agent can be designated as immunochemotherapy.

It is particularly preferred that the reference blood sample is isolated from the (same) patient in a time period of between 1 day and immediately prior to the administration of said drug and/or prior to the administration of another form of therapeutic treatment (than the administration of a drug). It is particularly more preferred that the reference blood sample is isolated from the (same) patient in a time period of between 0.5 day and immediately prior to the administration of said drug and/or prior to the administration of another form of therapeutic treatment (than the administration of a drug). For example, the reference blood sample is isolated from the (same) patient immediately, 10, 20, 30, 40, 50 minutes, 1, 2, 3, 4, 5, 6, 7, 8, 9, 10, 11, 12, 13, 14, 15, 16, 17, 18, 19, 20, 21, 22, 23 hour(s), or 1 day prior to the administration of said drug and/or prior to the administration of another form of therapeutic treatment (than the administration of a drug).

In one more preferred embodiment,
the level of the RNA molecule below the reference level indicates that the patient responds to said treatment of lung cancer,
the level of the RNA molecule comparable with the reference level indicates that the patient does not respond to said treatment of lung cancer, or
the level of the RNA molecule above the reference level indicates that the patient does not respond to said treatment of lung cancer.

Preferably, the level of the RNA molecule is at least 0.6-fold or 0.7-fold, more preferably at least 0.8-fold or 0.9-fold, even more preferably at least 1.2-fold or 1.5-fold, and still even more preferably at least 2.0-fold or 3.0-fold above/below the reference level. For example, the level of the RNA molecule is at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold above/below the reference level.

"Comparable" in the above context preferably means that the level varies between 0 and < 20%, specifically between 0 and < 10%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Comparable" in this respect may also mean that the detected level variation is within the accuracy of a measurement. The accuracy of a measurement depends on the measurement method used. Preferably, the level is constant over time.

If the therapeutic treatment encompasses the administration of a drug and the patient responds to said treatment, the drug administration may be continued. In particular, the dose of the drug may be reduced and/or the administration interval of the drug can be prolonged. Alternatively, the drug administration may be stopped. If the therapeutic treatment encompasses the administration of a drug and the patient does not respond to said treatment, the dose of the drug may be increased, the administration interval of the drug can be shortened, and/or the drug may be changed. Alternatively, the therapy mode may be changed, e.g. from the administration of a drug to surgery, radiotherapy, laser therapy, and hyperthermia therapy.

If the therapeutic treatment encompasses another therapeutic treatment than the administration of a drug and the patient responds to said treatment, the therapeutic treatment may be continued, the number of treatments may be reduced, or the intensity of the therapeutic treatments may be reduced. If the therapeutic treatment encompasses another therapeutic treatment than the administration of a drug and the patient does not respond to said treatment, the number of treatments may be increased and/or the intensity of the therapeutic treatments may be increased. Alternatively, the therapy mode may be changed.

Preferably, the drug is selected from the group consisting of an immunomodulatory agent, a chemotherapeutic agent, an immunotherapeutic agent, an immunosuppressive agent, a radiotherapeutic agent, an oncolytic virus, a vaccine, and an antibody. As mentioned above, the drug can also be a drug combination of at least two different medicaments. Thus, the patient may be a patient to whom at least once a chemotherapeutic agent and an immunotherapeutic agent has been administered or had been administered.

More preferably,
(i) the immunomodulatory agent is selected from the group consisting of a checkpoint inhibitor, preferably a checkpoint inhibitor targeting PD-1, PD-L1, PD-L2, CTLA-4 or intrinsic checkpoint blockades,
(ii) the chemotherapeutic agent is selected from the group consisting of an alkylating agent, an antimetabolite, folinic acid, a folate antagonist, a mitotic inhibitor, an anthracyclin, a topoisomerase inhibitor, a signal transduction inhibitor, an inhibitor of angiogenesis, and an inhibitor of histone deacetylase, or
(iii) immunotherapeutic agent is selected from the group consisting of a cytokine, an antibody, an antigen-presenting cell, or a chimeric antigen receptor T cell.

Preferably, the other form of therapeutic treatment is selected from the group consisting of surgery, radiotherapy, laser therapy, and hyperthermia therapy.

Preferably, the lung cancer is non-small-cell lung carcinoma (NSCLC) or small-cell lung carcinoma (SCLS). More preferably, the lung cancer is non-small-cell lung carcinoma (NSCLC).

The data in **FIGURE 7** clearly show that the above-mentioned RNA molecule allows to determine whether a patient responds to a therapeutic treatment of cancer/anti-cancer therapy or not. Specifically, the therapeutic treatment of cancer/anti-cancer therapy is chemotherapy or immunochemotherapy. In **FIGURE 7****,** the distribution of the above-mentioned RNA molecule in lung cancer samples before and after anti-cancer therapy of a patient is shown. The clinical assessments by the oncologist at the time of the follow-up visit (after therapy) summarize the development of the disease (since the previous visit) as either remission (disease improving), stable disease (no change), and progression (disease worsening). The level of the above-mentioned RNA molecule mirrors this assessment by (i) significantly decreasing during remission, which indicates that the patient responds to the treatment of lung cancer, (ii) significantly increasing during progression, which indicates that the patient does not respond to the treatment of lung cancer, and (iii) remaining constant during stable disease periods (no significant difference), which indicates that the patient does not respond to the treatment of lung cancer.

In a fourth aspect, the present invention relates to a (an *in vitro*) method of determining the risk for a relapse of lung cancer in a patient comprising the step of:
determining the level of an RNA molecule in a blood sample from a patient, wherein the RNA molecule comprises a nucleotide sequence according to SEQ ID NO: 1.

It is preferred that the patient had received a therapeutic treatment of lung cancer. Particularly, the patient is in remission (i.e. having no symptoms or signs of disease).

It is further preferred that said treatment involved the administration of
a drug for lung cancer, and/or
another form of therapeutic treatment of lung cancer.

The drug can also be a drug combination of at least two different medicaments, e.g. a combination of a chemotherapeutic agent and an immunotherapeutic agent. The administration of a chemotherapeutic agent in combination with an immunotherapeutic agent can be designated as immunochemotherapy.

The way of administration may be oral, nasal, rectal, parenteral, or topical. Parental administration includes subcutaneous, intracutaneous, intramuscular, intravenous or intraperitoneal administration.

It is also preferred that the blood sample is isolated after the therapeutic treatment of lung cancer.

In one embodiment, the level of the RNA molecule is compared to a reference level of said RNA molecule. Thus, in one particular embodiment, the present invention relates to a (an *in vitro*) method of determining the risk for a relapse of lung cancer in a patient comprising the steps of:
(i) determining the level of an RNA molecule in a blood sample from a patient (isolated after the therapeutic treatment of lung cancer), and
(ii) comparing the level of the RNA molecule to a reference level of said RNA molecule,
wherein the RNA molecule comprises a nucleotide sequence according to SEQ ID NO: 1, is a fragment thereof, or has a sequence having at least 80%, preferably at least 85%, more preferably at least 90%, and most preferably at least 95% or 99%, e.g. at least 80, 81, 82, 83, 84, 85, 86, 87, 88, 89, 90, 91, 92, 93, 94, 95, 96, 97, 98, or 99%, sequence identity thereto.

The above comparison allows to evaluate whether a patient in remission (i.e. having no symptoms or signs of disease) has a risk of getting sick again. A recurrence occurs when the lung cancer comes back after treatment. This can happen weeks, months, or even years after the primary or original lung cancer was treated. It is impossible to know for sure if the lung cancer will recur. The chance of recurrence depends on the type of primary cancer. Cancer recurs because small areas of cancer cells can remain in the body after treatment. Over time, these cells may multiply and grow large enough to cause symptoms or for tests to find them.

The reference level may be any level which allows to determine whether a patient in remission (i.e. having no symptoms or signs of disease) has a risk of getting sick again. It may be obtained from (control) subjects (i.e. healthy subjects or subjects different from the patient to be tested such as successfully treated lung cancer patients).

In one preferred embodiment, the reference level is the level of the RNA molecule determined empirically by measuring a number of reference blood samples from healthy subjects, and/or
successfully treated lung cancer patients.

For example, the reference level is determined from at least 2, at least 10, at least 50, at least 100, at least 200, at least 300, at least 400, at least 500, at least 1000, at least 1500, at least 2000 at least 5000 reference samples from healthy subjects.

For example, the reference level is determined from at least 2, at least 10, at least 50, at least 100, at least 200, at least 300, at least 400, at least 500, at least 1000, at least 1500, at least 2000 at least 5000 reference samples from successfully treated lung cancer patients.

Specifically, the reference level is an average reference level. It is determined by measuring reference levels of control subjects (e.g. healthy subjects or successfully treated lung cancer patients) and calculating the "average" value (e.g. mean, median or modal value) thereof. It is preferred that the reference blood sample is from the same source (e.g. blood cells, serum, or plasma) than the blood sample isolated from the patient to be tested. It is further preferred that the reference level is obtained from control subjects (e.g. healthy subjects or successfully treated lung cancer patients) of the same gender (e.g. female or male) and/or of a similar age/phase of life (e.g. adults or elderly) than the patient to be tested. Particularly, the reference level represents an average value of the RNA molecule in a healthy population and/or the reference level represents an average value of the RNA molecule in a population of successfully treated lung cancer patients. More particularly, the population group of successfully treated lung cancer patients includes subjects of a specific lung cancer stage, e.g. I, II, III or IV, or subjects of all lung cancer stages, e.g. I, II, III, and IV (mixed population, specifically equally represented).

In one more preferred embodiment, the level of the RNA molecule above the reference level indicates a risk of the patient for a relapse of lung cancer.

Preferably, the level of the RNA molecule is at least 0.6-fold or 0.7-fold, more preferably at least 0.8-fold or 0.9-fold, even more preferably at least 1.2-fold or 1.5-fold, and still even more preferably at least 2.0-fold or 3.0-fold above the reference level. For example, the level of the RNA molecule is at least 0.6-fold, at least 0.7-fold, at least 0.8-fold, at least 0.9-fold, at least 1.0-fold, at least 1. 1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold above the reference level.

In one alternatively or additionally preferred embodiment, the reference level represents the minimal level of the RNA molecule achievable/achieved in the patient by the therapeutic treatment of lung cancer.

In one more preferred embodiment, the level of the RNA molecule above the reference level indicates a risk of the patient for a relapse of lung cancer.

Preferably, the level of the RNA molecule is at least 0.6-fold or 0.7-fold, more preferably at least 0.8-fold or 0.9-fold, even more preferably at least 1.2-fold or 1.5-fold, and still even more preferably at least 2.0-fold or 3.0-fold above the reference level. For example, the level of the RNA molecule is at least 0.6-fold, at least 0.7-fold, at least 0.8-fold, at least 0.9-fold, at least 1.0-fold, at least 1. 1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold above the reference level.

Said above mentioned "minimal level" may be a single value achievable/achieved in the patient by the therapeutic treatment of lung cancer, or may be an average value of a number of values achievable/achieved in the patient during the therapeutic treatment period of lung cancer.

As already mentioned above, the reference level may be any level which allows to determine whether a patient in remission (i.e. having no symptoms or signs of disease) has a risk of getting sick again. It may be obtained from (control) subjects (i.e. healthy subjects or subjects different from the patient to be tested such as successfully treated lung cancer patients). It may also be obtained from the same individual.

Accordingly, in one alternative or additional embodiment, the determining comprises determining the level of the RNA molecule in a blood sample obtained from a patient at a first point in time after therapeutic treatment of lung cancer and in at least one further blood sample obtained from the (same) patient at a later point in time and comparing said levels determined at the different time points. Thus, in one particular embodiment, the present invention relates to a (an *in vitro)* method of determining the risk for a relapse of lung cancer in a patient comprising the steps of:
(i) determining the level of the RNA molecule in a blood sample obtained from a patient at a first point in time after therapeutic treatment of lung cancer and in at least one further blood sample obtained from the (same) patient at a later point in time, and
(ii) comparing said levels determined at the different time points,
wherein the RNA molecule comprises a nucleotide sequence according to SEQ ID NO: 1.

The above comparison allows to evaluate whether a patient in remission (i.e. having no symptoms or signs of disease) has a risk of getting sick again. A recurrence occurs when the lung cancer comes back after treatment. This can happen weeks, months, or even years after the primary or original lung cancer was treated. It is impossible to know for sure if the lung cancer will recur. The chance of recurrence depends on the type of primary cancer. Cancer recurs because small areas of cancer cells can remain in the body after treatment. Over time, these cells may multiply and grow large enough to cause symptoms or for tests to find them.

In one preferred embodiment, the level of the RNA molecule which increases over time indicates a risk of the patient for a relapse of lung cancer.

Preferably, the increase is at least 0.6-fold or 0.7-fold, more preferably at least 0.8-fold or 0.9-fold, even more preferably at least 1.2-fold or 1.5-fold, and still even more preferably at least 2.0-fold or 3.0-fold over time.

For example, the increase may be at least 0.6-fold, at least 0.7-fold, at least 0.8-fold, at least 0.9-fold, at least 1.0-fold, at least 1. 1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2. 1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold over time.

The time period between the first point in time and the later point(s) in time preferably amounts to at least 1 day, at least 2 days, at least 3 days, at least 4 days, at least 5 days, at least 6 days, at least 7 days (1 week), at least 2 weeks, at least 3 weeks, at least 4 weeks, at least 1 month, at least 2 months, at least 3 months, at least 4 months, at least 5 months, at least 6 months, at least 7 months, at least 8 months, at least 9 months, at least 10 months, at least 11 months, at least 12 months (1 year), at least 24 months (2 years), at least 3 years, at least 4 years, at least 5 years, at least 6 years, at least 7 years, at least 8 years, at least 9 years, or at least 10 years. For example, the individual may be routinely checked, e.g. once or twice a year. The patient may be (re)tested at 2, 3, 4, 5, 6 7, 8, 9, or 10 time points (first point in time and further point(s) in time).

Preferably, the drug is selected from the group consisting of an immunomodulatory agent, a chemotherapeutic agent, an immunotherapeutic agent, an immunosuppressive agent, a radiotherapeutic agent, an oncolytic virus, a vaccine, and an antibody. As mentioned above, the drug can also be a drug combination of at least two different medicaments. Thus, the patient may be a patient to whom at least once a chemotherapeutic agent and an immunotherapeutic agent has been administered or had been administered.

More preferably,
(i) the immunomodulatory agent is selected from the group consisting of a checkpoint inhibitor, preferably a checkpoint inhibitor targeting PD-1, PD-L1, PD-L2, CTLA-4 or intrinsic checkpoint blockades,
(ii) the chemotherapeutic agent is selected from the group consisting of an alkylating agent, an antimetabolite, folinic acid, a folate antagonist, a mitotic inhibitor, an anthracyclin, a topoisomerase inhibitor, a signal transduction inhibitor, an inhibitor of angiogenesis, and an inhibitor of histone deacetylase, or
(iii) immunotherapeutic agent is selected from the group consisting of a cytokine, an antibody, an antigen-presenting cell, or a chimeric antigen receptor T cell.

Preferably, the other form of therapeutic treatment is selected from the group consisting of surgery, radiotherapy, laser therapy, and hyperthermia therapy.

Preferably, the lung cancer is non-small-cell lung carcinoma (NSCLC) or small-cell lung carcinoma (SCLS). More preferably, the lung cancer is non-small-cell lung carcinoma (NSCLC).

In a fifth aspect, the present invention relates to a (an *in vitro)* method of detecting a minimal residual disease (MRD) in a patient having lung cancer comprising the step of: determining the level of an RNA molecule in a blood sample from a patient, wherein the RNA molecule comprises a nucleotide sequence according to SEQ ID NO: 1.

It is preferred that the patient has received or had received a therapeutic treatment of lung cancer.

It is further preferred that said treatment involves or involved the administration of
a drug for lung cancer, and/or
another form of therapeutic treatment of lung cancer.

The drug can also be a drug combination of at least two different medicaments, e.g. a combination of a chemotherapeutic agent and an immunotherapeutic agent. The administration of a chemotherapeutic agent in combination with an immunotherapeutic agent can be designated as immunochemotherapy.

The way of administration may be oral, nasal, rectal, parenteral, or topical. Parental administration includes subcutaneous, intracutaneous, intramuscular, intravenous or intraperitoneal administration.

It is also preferred that the blood sample is isolated during or after the therapeutic treatment of lung cancer.

In one embodiment, the level of the RNA molecule is compared to a reference level of said RNA molecule. Thus, in one particular embodiment, the present invention relates to a (an *in vitro)* method of detecting a minimal residual disease (MRD) in a patient having lung cancer comprising the steps of:
(i) determining the level of an RNA molecule in a blood sample from a patient (isolated during or after therapeutic treatment of lung cancer), and
(ii) comparing the level of the RNA molecule to a reference level of said RNA molecule, wherein the RNA molecule comprises a nucleotide sequence according to SEQ ID NO: 1.

The above comparison allows to evaluate whether there is minimal lung cancer left in the patient. In particular, the detection of a minimal residual disease in a patient is indicative for the presence of small numbers of cancer cells that remained in the patient during treatment, or after treatment when the patient is in remission (no symptoms or signs of disease). It is the major cause of relapse in cancer. In cancer treatment, MRD testing has several important roles: determining whether treatment has eradicated the cancer or whether traces remain, comparing the efficacy of different treatments, monitoring patient remission status as well as detecting recurrence of cancer, and choosing the treatment that will best meet those needs.

The reference level may be any level which allows to determine whether a patient suffers from a minimal residual disease. It may be obtained from (control) subjects (i.e. healthy subjects or subjects different from the patient to be tested such as successfully treated lung cancer patients).

In one preferred embodiment, the reference level is the level of the RNA molecule determined empirically by measuring a number of reference blood samples from healthy subjects, and/or
successfully treated lung cancer patients.

For example, the reference level is determined from at least 2, at least 10, at least 50, at least 100, at least 200, at least 300, at least 400, at least 500, at least 1000, at least 1500, at least 2000 at least 5000 reference samples from healthy subjects.

For example, the reference level is determined from at least 2, at least 10, at least 50, at least 100, at least 200, at least 300, at least 400, at least 500, at least 1000, at least 1500, at least 2000 at least 5000 reference samples from successfully treated lung cancer patients.

Specifically, the reference level is an average reference level. It is determined by measuring reference levels of control subjects (e.g. healthy subjects or successfully treated lung cancer patients) and calculating the "average" value (e.g. mean, median or modal value) thereof. It is preferred that the reference blood sample is from the same source (e.g. blood cells, serum, or plasma) than the blood sample isolated from the patient to be tested. It is further preferred that the reference level is obtained from control subjects (e.g. healthy subjects or successfully treated lung cancer patients) of the same gender (e.g. female or male) and/or of a similar age/phase of life (e.g. adults or elderly) than the patient to be tested. Particularly, the reference level represents an average value of the RNA molecule in a healthy population and/or the reference level represents an average value of the RNA molecule in a population of successfully treated lung cancer patients. More particularly, the population group of successfully treated lung cancer patients includes subjects of a specific lung cancer stage, e.g. I, II, III or IV, or subjects of all lung cancer stages, e.g. I, II, III, and IV (mixed population, specifically equally represented).

In one more preferred embodiment, the level of the RNA molecule above the reference level indicates that the patient suffers from a minimal residual disease.

Preferably, the patient suffering from a minimal residual disease is assigned to/eligible for adjuvant therapy. More preferably, the patient who is assigned to/eligible for adjuvant therapy is subsequently treated with said lung cancer therapy.

In one alterative or additional embodiment, the determining comprises determining the level of the RNA molecule in a blood sample obtained from a patient at a first point in time before therapeutic treatment of lung cancer and in at least one further blood sample obtained from the (same) patient at a later point in time after therapeutic treatment and comparing said levels determined at the different time points. Thus, in one particular embodiment, the present invention relates to a (an *in vitro)* method of detecting a minimal residual disease in a patient having lung cancer comprising the steps of:
(i) determining the level of the RNA molecule in a blood sample obtained from a patient at a first point in time before therapeutic treatment of lung cancer and in at least one further blood sample obtained from the (same) patient at a later point in time after therapeutic treatment, and
(ii) comparing said levels determined at the different time points,
wherein the RNA molecule comprises a nucleotide sequence according to SEQ ID NO: 1.

In one preferred embodiment, the level of the RNA molecule which is stable over time or does not decrease over time up to/under a threshold level (representing the minimal level of the RNA molecule achievable by the therapeutic treatment of lung cancer) indicates that the patient suffers from a minimal residual disease. The threshold level preferably represents the minimal level of the RNA molecule achievable by therapeutic treatment of lung cancer.

Preferably, the patient suffering from a minimal residual disease is assigned to/eligible for adjuvant therapy. More preferably, the patient who is assigned to/eligible for adjuvant therapy is subsequently treated with said lung cancer therapy.

The time period between the first point in time and the later point(s) in time preferably amounts to at least 1 day, at least 2 days, at least 3 days, at least 4 days, at least 5 days, at least 6 days, at least 7 days (1 week), at least 2 weeks, at least 3 weeks, at least 4 weeks, at least 1 month, at least 2 months, at least 3 months, at least 4 months, at least 5 months, at least 6 months, at least 7 months, at least 8 months, at least 9 months, at least 10 months, at least 11 months, at least 12 months (1 year), at least 24 months (2 years), at least 3 years, at least 4 years, at least 5 years, at least 6 years, at least 7 years, at least 8 years, at least 9 years, or at least 10 years. For example, the individual may be routinely checked, e.g. once or twice a year. The patient may be (re)tested at 2, 3, 4, 5, 6 7, 8, 9, or 10 time points (first point in time and further point(s) in time).

Preferably, the drug is selected from the group consisting of an immunomodulatory agent, a chemotherapeutic agent, an immunotherapeutic agent, an immunosuppressive agent, a radiotherapeutic agent, an oncolytic virus, a vaccine, and an antibody. As mentioned above, the drug can also be a drug combination of at least two different medicaments. Thus, the patient may be a patient to whom at least once a chemotherapeutic agent and an immunotherapeutic agent has been administered or had been administered.

More preferably,
(i) the immunomodulatory agent is selected from the group consisting of a checkpoint inhibitor, preferably a checkpoint inhibitor targeting PD-1, PD-L1, PD-L2, CTLA-4 or intrinsic checkpoint blockades,
(ii) the chemotherapeutic agent is selected from the group consisting of an alkylating agent, an antimetabolite, folinic acid, a folate antagonist, a mitotic inhibitor, an anthracyclin, a topoisomerase inhibitor, a signal transduction inhibitor, an inhibitor of angiogenesis, and an inhibitor of histone deacetylase, or
(iii) immunotherapeutic agent is selected from the group consisting of a cytokine, an antibody, an antigen-presenting cell, or a chimeric antigen receptor T cell.

Preferably, the other form of therapeutic treatment is selected from the group consisting of surgery, radiotherapy, laser therapy, and hyperthermia therapy.

Preferably, the lung cancer is non-small-cell lung carcinoma (NSCLC) or small-cell lung carcinoma (SCLS). More preferably, the lung cancer is non-small-cell lung carcinoma (NSCLC).

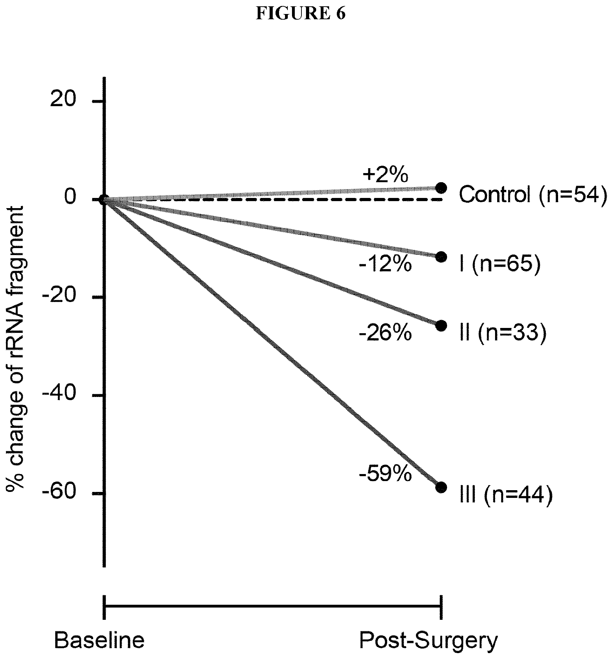

**FIGURE 6** shows the reduction of the above-mentioned RNA molecule after lung cancer surgery (depending on stages). Therefore, the above-mentioned RNA molecule level is tumor-associated. Thus, the determination of the above-mentioned RNA molecule level allows to assess a minimal residual disease in a patent having cancer, e.g. after surgery, likely as therapy control.

In the methods of the first to fifth aspect, the patient is preferably a mammal, more preferably a human or a rodent, even more preferably a human.

In the methods of the first to fifth aspect, the blood sample is preferably whole blood or a blood fraction. In particular the blood fraction is selected from the group consisting of a blood cell fraction and plasma or serum. More preferably, the blood fraction is selected from the group consisting of a blood cell fraction, plasma, and serum. The blood cell fraction may comprise erythrocytes, leukocytes, and/or thrombocytes. Even more preferably, the blood cell fraction is a fraction of leukocytes or the blood cell fraction a mixture of erythrocytes, leukocytes, and thrombocytes.

In the methods of the first to fifth aspect, the level of the RNA molecule is preferably determined by sequencing, preferably next generation sequencing (e.g. ABI SOLID, Illumina Genome Analyzer, Roche 454 GS FL, BGISEQ), nucleic acid hybridization (e.g. microarray or beads), nucleic acid amplification (e.g. PCR, RT-PCR, qRT-PCR, or high-throughput RT-PCR), polymerase extension, mass spectrometry, flow cytometry (e.g. LUMINEX), or any combination thereof. Specifically, the level of the RNA molecule is the expression level of said RNA molecule.

In a sixth aspect, the present invention relates to the (*in vitro*) use of an RNA molecule for diagnosing lung cancer in a patient, monitoring the course of lung cancer in a patient, determining whether a patient responds to a therapeutic treatment of lung cancer, determining the risk for a relapse of lung cancer in a patient, and/or detecting a minimal residual disease (MRD) in a patient having lung cancer,
wherein the RNA molecule comprises a nucleotide sequence according to SEQ ID NO: 1.

For the above-mentioned use, the level the RNA molecule is determined/analyzed in a blood sample from the patient.

Preferably, the blood sample is whole blood or a blood fraction. In particular the blood fraction is selected from the group consisting of a blood cell fraction and plasma or serum. More preferably, the blood fraction is selected from the group consisting of a blood cell fraction, plasma, and serum. The blood cell fraction may comprise erythrocytes, leukocytes, and/or thrombocytes. Even more preferably, the blood cell fraction is a fraction of leukocytes or the blood cell fraction a mixture of erythrocytes, leukocytes, and thrombocytes.

Preferably, the lung cancer is non-small-cell lung carcinoma (NSCLC) or small-cell lung carcinoma (SCLS). More preferably, the lung cancer is non-small-cell lung carcinoma (NSCLC).

As to other preferred embodiments, it is referred to the first to fourth aspect of the present invention.

In a seventh aspect, the present invention relates to the (*in vitro*) use of a kit for diagnosing lung cancer in a patient, monitoring the course of lung cancer in a patient, determining whether a patient responds to a therapeutic treatment of lung cancer, determining the risk for a relapse of lung cancer in a patient, and/or detecting a minimal residual disease (MRD) in a patient having lung cancer, wherein said kit comprises:
means for determining the level of an RNA molecule in a blood sample from a patient, wherein the RNA molecule comprises a nucleotide sequence according to SEQ ID NO: 1.

More specifically, the kit comprises
means for carrying out next generation sequencing (NGS),
at least one polynucleotide (probe) for detecting the RNA molecule,
at least one primer (e.g. a primer pair) for binding the RNA molecule, and/or
at least one antibody capable of binding a hybrid of said at least one polynucleotide (probe) and said RNA molecule.

The at least one polynucleotide (probe) may be part of a microarray/biochip or may be attached to beads of a beads-based multiplex system. The at least one polynucleotide (primer, primer pair) may be part of a RT-PCR system, a PCR-system, or a next generation sequencing system. Said means may further comprise a microarray, a RT-PCT system, a PCR-system, a flow cytometer, a Luminex system, and/or a next generation sequencing system.

In one preferred embodiment, the kit further comprises instructions on how to carry out the methods according to the first to fifth aspect of the present invention.

In one another preferred embodiment, the kit is useful for conducting the methods according to the first to fifth aspect of the present invention.

The kit may further comprise
a container, and/or
a data carrier.

The data carrier may be a non-electronical data carrier, e.g. a graphical data carrier such as an information leaflet, an information sheet, a bar code or an access code, or an electronical data carrier such as a floppy disk, a compact disk (CD), a digital versatile disk (DVD), a microchip or another semiconductor-based electronical data carrier. The access code may allow the access to a database, e.g. an internet database, a centralized, or a decentralized database. The access code may also allow access to an application software that causes a computer to perform tasks for computer users or a mobile app which is a software designed to run on smartphones and other mobile devices.

Said data carrier may further comprise at least one reference, e.g. the reference level of the level of the RNA molecule determined herein or a threshold level. In case that the data carrier comprises an access-code which allows the access to a database, said at least one reference, e.g. said reference level or threshold level may be deposited in this database.

The data carrier may also comprise information or instructions on how to carry out the method according to the first to fifth aspect of the present invention.

The kit may also comprise materials desirable from a commercial and user standpoint including a buffer(s), a reagent(s) and/or a diluent(s) for determining the level mentioned above.

Preferably, the blood sample is whole blood or a blood fraction. In particular the blood fraction is selected from the group consisting of a blood cell fraction and plasma or serum. More preferably, the blood fraction is selected from the group consisting of a blood cell fraction, plasma, and serum. The blood cell fraction may comprise erythrocytes, leukocytes, and/or thrombocytes. Even more preferably, the blood cell fraction is a fraction of leukocytes or the blood cell fraction a mixture of erythrocytes, leukocytes, and thrombocytes.

Preferably, the lung cancer is non-small-cell lung carcinoma (NSCLC) or small-cell lung carcinoma (SCLS). More preferably, the lung cancer is non-small-cell lung carcinoma (NSCLC).

### BRIEF DESCRIPTION OF THE FIGURES

The following Figures are merely illustrative of the present invention and should not be construed to limit the scope of the invention as indicated by the appended claims in any way.
**Figure 1****:** Shows boxplots of log2(1+RPM) expression per tumor stage. The 28S ribosomal RNA (rRNA) fragment GCCGCCGGUGAAAUACCACUAC (SEQ ID NO: 1) was expressed more highly with increasing tumor stage in a clinical trial on lung cancer patients.
**Figure 2****:** Histograms of log2(1+RPM) expression of Control group and CaseLC group plotted with Gaussian kernel density estimates as smoothend curves. When grouping all lung cancer patients (CaseLC), the histogram of log2-RPM expression shows a clear increase compared to controls.
**Figure 3****:** Cohen's d effect size (diagnosis) vs logistic regression model coefficients for features with non-zero model coefficients.
**Figure 4****:** DESeq2's adjusted p-values over log2 fold change (diagnosis) for features with non-zero model coefficients.
**Figure 5****:** Binary classification (diagnosis) model performance on held-out test set. Performance is given as areas under the ROC curve (AUC).
**Figure 6****:** Shows the reduction of the 28S ribosomal RNA (rRNA) fragment after surgery (depending on stages).
**Figure 7****:** Shows the distribution of 28S ribosomal RNA (rRNA) fragment GCCGCCGGUGAAAUACCACUAC (SEQ ID NO: 1) expression in clinical lung cancer samples before and after anti-cancer therapy as box plots. Clinical assessments by the oncologist at the time of the follow-up visit (after therapy) summarize the development of the disease (since the previous visit) as either remission (disease improving), stable disease (no change), and progression (disease worsening). 28S ribosomal RNA (rRNA) fragment expression mirrors this assessment by significantly decreasing during remission, significantly increasing during progression, and remaining constant during stable disease periods (no significant difference). The statistical quantification of this difference was obtained via paired T-tests with a two-sided alternative hypothesis (there is a change in expression after treatment vs before). P-values and Cohen's d signed effect sizes (signs indicating the direction of change) are given for each test as well as the sample size N. P-values are categorized by significance (lower P-value indicating greater significance) using the following convention: ns (not significant): P>0.05; *: P ≤ 0.05; **: P ≤ 0.01; ***: P ≤ 0.001; ****: P ≤ 0.0001. Results are given for (a) all therapy types combined, (b) only cases where chemotherapy was given, potentially in combination with other therapies, and (c) only cases where immuno-chemo therapy was given, potentially combined with other therapies.

### EXAMPLES

The examples given below are for illustrative purposes only and do not limit the invention described above in any way.

The present inventors could show that the determination of the 28S ribosomal RNA (rRNA) fragment GCCGCCGGUGAAAUACCACUAC (SEQ ID NO: 1) level in a blood sample of a patient allows the diagnosis and monitoring of lung cancer. The present inventors could also show that the determination of the 28S ribosomal RNA (rRNA) fragment level in a blood sample of a patient allows to assess the success of an anti-lung cancer therapy. Specifically, the present inventors could show that the determination of the 28S ribosomal RNA (rRNA) fragment GCCGCCGGUGAAAUACCACUAC (SEQ ID NO: 1) level in a blood sample allows to assess whether a patient is in remission after anti-lung cancer therapy, whether a patient is stable (no improvement or deterioration of lung cancer), or whether lung cancer is further progressing in the patient (deterioration of lung cancer).

### 1. Experiment

### Materials & Methods

A small-RNA next generation sequencing (NGS) dataset of 1427 patients was used, leaving out only those patients with an unclear diagnosis or low QC metrics (based on in-house spike-ins and total read counts). Of over 1.8 M unique RNA sequences detected in this dataset, 18312 sequences remained after removing those with a maximum log2(1+RPM) expression below 10, where RPM stands for Reads Per Million.

These patients were first diagnosis-balanced (via under-sampling the majority group) within each of two clinics to avoid any batch effect due to potential differences between the clinics. The resulting balanced dataset therefore had equal counts for the Control (non-tumor lung disease) and the CaseLC (lung cancer) groups.

A uniformly sampled test set of 25% of patients was removed, whereas the remaining 75% of patients were used to train a logistic regression classifier model. Training samples were further reduced to the 10000 most variable sequences (features) within each NGS-pooling group, thus reducing the influence of a major batch variable. Furthermore, any sequence detected in less than 20% of the training samples was removed. The remaining 9132 features were standard-scaled and used as the input for an L1-penalized logistic regression model with a penalty of 20.

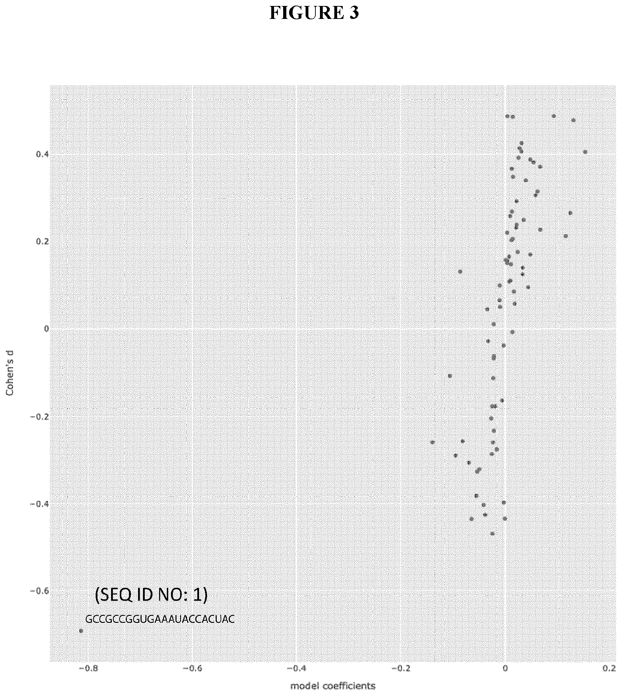

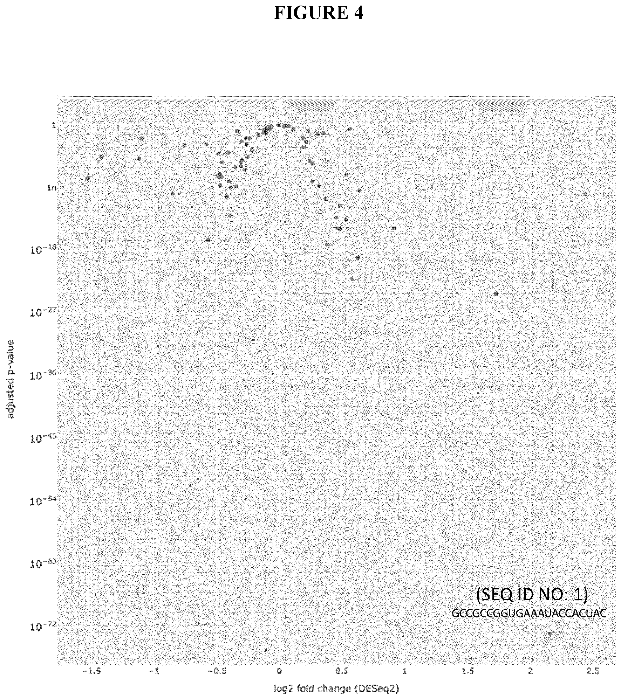

After fitting the model coefficients to the discrimination between Control and CaseLC samples, only 78 features remained with non-zero coefficients. These coefficients are somewhat correlated with Cohen's d effect size (r=0.59; see **FIGURE 3****).** DESeq2 is a standard software tool to calculate significant differential expression for RNA-seq data. Adjusted p-values and log2 fold changes between Control and CaseLC groups is given in **FIGURE 4****.**

### Results

The strongest outlier in all of Cohen's d, model coefficients, as well as DESeq2's adjusted p-values was the 28S ribosomal RNA (rRNA) fragment GCCGCCGGUGAAAUACCACUAC (SEQ ID NO: 1).

This RNA molecule was found to be expressed more highly with increasing tumor stage in a clinical trial on lung cancer patients with non-tumor lung disease patients as controls (see **FIGURE 1**). When grouping all lung cancer patients (CaseLC), the histogram of log2-RPM expression shows a clear increase compared to controls (see **FIGURE 2**).

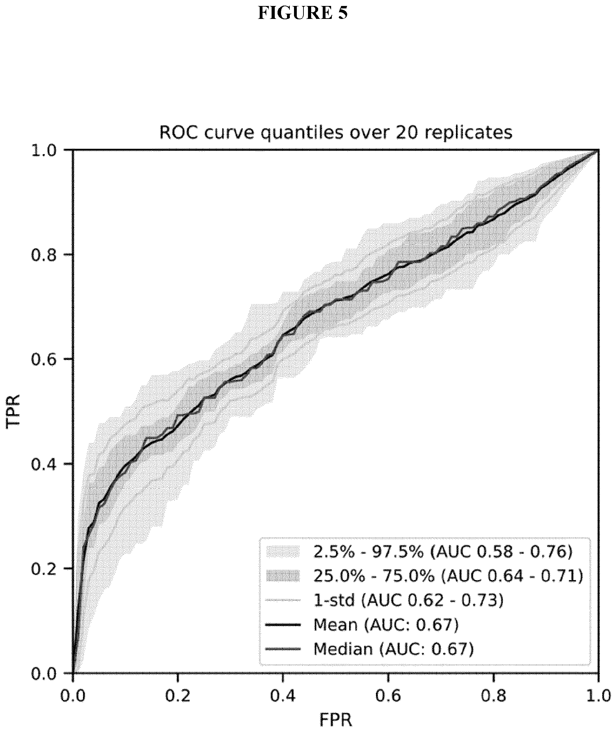

Over the entire test set, AUC was 0.67 and, thus, much higher when compared to an AUC of 0.46 when shuffling the diagnosis labels (indicating no better performance than random guessing (see **FIGURE 5**).

Exact patient numbers used for training and testing (listed per stage) are given in **TABLE 1** below:

**TABLE 1: Number of patients used as controls or cases (split by tumor stage) per training and test set.**

| # patients | Training set | Test set |
|---|---|---|
| Control | 494 | 189 |
| Stage I cancer | 100 | 36 |
| Stage II cancer | 66 | 13 |
| Stage III cancer | 161 | 49 |
| Stage IV cancer | 204 | 54 |

**FIGURE 6** further shows the reduction of the 28S ribosomal RNA (rRNA) fragment after lung cancer surgery (depending on stages). Thus, the 28S ribosomal RNA (rRNA) fragment level is tumor-associated. The determination of the 28S ribosomal RNA (rRNA) fragment level may also be used to determine a minimal residual disease in a patent having lung cancer, e.g. after surgery, likely as therapy control.

### 2. Experiment

### Materials & Methods

For a subset of lung cancer patients as described in experiment 1 above, follow-up visits were accompanied with blood draws to monitor their response to various anti-cancer treatments deemed appropriate by the oncologists. These follow-up samples for treated in the same way as baseline samples (see above). Multiple follow-ups per patient were possible.

Consecutive visits of a patient were paired regardless of the number of preceding visits. These paired visits were then categorized according to the ancologist's assessment into "progression", "stable disease", or "progression". The expression of the RNA (SEQ ID NO. 1) was obtained for each patient visit as log2(1+RPM). Paired T-tests were performed on expression values within each category with a two-sided alternative hypothesis stating: "there is a significant difference in expression between the first and second visit in each pair of visits". Cohen's d effect sizes and T-tests on paired samples were computed using the pingouin statistics software package (https://pingouin-stats.org).

**TABLE 2: Patient counts for pairs of consecutive visits, including the initial visit (baseline,BL) and numbered follow-visits (F-..). E.g. "BL F-01" is the interval between the pre-treatment visit (BL) and the first follow-up visit (F-01) during which anti-cancer therapies have taken place. Numbers indicate the order of visits, but the duration between visits is variable.**

| **Pair of consecutive visits** | **Patient count** |
|---|---|
| BL_F-01 | 440 |
| F-01_F-02 | 258 |
| F-02_F-03 | 149 |
| F-03_F-04 | 99 |
| F-04_F-05 | 72 |
| F-05_F-06 | 51 |
| F-06_F-07 | 35 |
| F-07_F-08 | 24 |
| F-08_F-09 | 10 |
| F-09_F-10 | 7 |
| F-10_F-11 | 1 |
| F-11_F-12 | 1 |

### Results

The distribution of 28S ribosomal RNA (rRNA) fragment GCCGCCGGUGAAAUACCACUAC (SEQ ID NO: 1) expression in clinical lung cancer samples before and after anti-cancer therapy has further been evaluated. The results are shown in **FIGURE 7** as box plots. Clinical assessments by the oncologist at the time of the follow-up visit (after therapy) summarize the development of the disease (since the previous visit) as either remission (disease improving), stable disease (no change), and progression (disease worsening). 28S ribosomal RNA (rRNA) fragment expression mirrors this assessment by significantly decreasing during remission, significantly increasing during progression and remaining constant during stable disease periods (no significant difference). The statistical quantification of this difference was obtained via paired T-tests with a two-sided alternative hypothesis (there is a change in expression after treatment vs before). P-values and Cohen's d signed effect sizes (signs indicating the direction of change) are given for each test as well as the sample size N. P-values are categorized by significance (lower P-value indicating greater significance) using the following convention: ns (not significant): P>0.05; *: P ≤ 0.05; **: P ≤ 0.01; ***: P ≤ 0.001; ****: P ≤ 0.0001. Results are given for (a) all therapy types combined, (b) only cases where chemotherapy was given, potentially in combination with other therapies, and (c) only cases where immuno-chemo therapy was given, potentially combined with other therapies. In this respect, it should be noted that the box-plots for all therapy types combined include the patient data after chemotherapy, radiotherapy, immunotherapy, immunochemotherapy, surgery, targeted therapy, or no therapy ("wait and see" category).

## Claims

1. A method of diagnosing lung cancer in a patient comprising the step of:
determining the level of an RNA molecule in a blood sample from a patient, wherein the RNA molecule comprises a nucleotide sequence according to SEQ ID NO: 1.

2. The method of claim 1,
wherein the level of the RNA molecule is compared to a reference level of said RNA molecule,
wherein preferably the reference level is the level of the RNA molecule determined empirically by measuring a number of reference blood samples from healthy subjects, and/or
subjects having lung cancer, and
wherein more preferably
the reference level is the level of the RNA molecule determined empirically by measuring a number of reference blood samples from healthy subjects, and wherein the level of the RNA molecule above the reference level indicates that the patient suffers from lung cancer, and/or
the reference level is the level of the RNA molecule determined empirically by measuring a number of reference blood samples from subjects having lung cancer, and wherein the level of the RNA molecule comparable with the reference level indicates that the patient suffers from lung cancer.

3. A method of monitoring the course of lung cancer in a patient comprising the step of:
determining the level of an RNA molecule in a blood sample from a patient, wherein the RNA molecule comprises a nucleotide sequence according to SEQ ID NO: 1.

4. The method of claim 3, wherein said monitoring comprises determining the level of the RNA molecule in a blood sample obtained from a patient at a first point in time and in at least one further blood sample obtained from the (same) patient at a later point in time and comparing said levels determined at the different time points.

5. The method of claim 4, wherein the level of the RNA molecule which
(i) increases over time indicates that the patient has developed lung cancer or that lung cancer is worsening in the patient,
(ii) does not change over time indicates that the patient is stable or that lung cancer is not progressing in the patient, or
(iii) decreases over time indicates that lung cancer is improving in the patient.

6. A method of determining whether a patient responds to a therapeutic treatment of lung cancer comprising the step of:
determining the level of an RNA molecule in a blood sample from a patient, wherein the RNA molecule comprises a nucleotide sequence according to SEQ ID NO: 1.

7. The method of claim 6, wherein the patient is a patient to whom at least once
a drug to be used in said therapeutic treatment, and/or
another form of therapeutic treatment
has been administered or had been administered.

8. A method of determining the risk for a relapse of lung cancer in a patient comprising the step of:
determining the level of an RNA molecule in a blood sample from a patient, wherein the RNA molecule comprises a nucleotide sequence according to SEQ ID NO: 1.

9. The method of claim 8, wherein the patient had received a therapeutic treatment of lung cancer, wherein said treatment preferably involved the administration of
a drug for lung cancer, and/or
another form of therapeutic treatment of lung cancer.

10. A method of detecting a minimal residual disease in a patient having lung cancer comprising the step of:
determining the level of an RNA molecule in a blood sample from a patient, wherein the RNA molecule comprises a nucleotide sequence according to SEQ ID NO: 1.

11. The method of claim 10, wherein the patient has received or had received a therapeutic treatment of lung cancer, wherein said treatment preferably involves or involved the administration of
a drug for lung cancer, and/or
another form of therapeutic treatment of lung cancer.

12. The method of any one of claims 1 to 11, wherein the lung cancer is non-small-cell lung carcinoma (NSCLC).

13. The method of any one of claims 1 to 12, wherein the blood sample is whole blood or a blood fraction.

14. Use of an RNA molecule for diagnosing lung cancer in a patient, monitoring the course of lung cancer in a patient, determining whether a patient responds to a therapeutic treatment of lung cancer, determining the risk for a relapse of lung cancer in a patient, and/or detecting a minimal residual disease in a patient having lung cancer,
wherein the RNA molecule comprises a nucleotide sequence according to SEQ ID NO: 1.

15. Use of a kit for diagnosing lung cancer in a patient, monitoring the course of lung cancer in a patient, determining whether a patient responds to a therapeutic treatment of lung cancer, determining the risk for a relapse of lung cancer in a patient, and/or detecting a minimal residual disease in a patient having lung cancer, wherein said kit comprises: means for determining the level of an RNA molecule in a blood sample from a patient, wherein the RNA molecule comprises a nucleotide sequence according to SEQ ID NO: 1.

## Patentansprüche

1. Verfahren zur Diagnose von Lungenkrebs bei einem Patienten, umfassend den Schritt: Bestimmen des Levels eines RNA-Moleküls in einer Blutprobe eines Patienten, wobei das RNA-Molekül eine Nukleotidsequenz gemäß SEQ ID NO: 1 umfasst.

2. Verfahren nach Anspruch 1,
wobei das Level des RNA-Moleküls mit einem Referenzlevel des RNA-Moleküls verglichen wird,
wobei bevorzugt
das Referenzlevel das Level des RNA-Moleküls ist, das empirisch durch Messung einer Anzahl von Referenzblutproben von
gesunden Probanden, und/oder
Probanden mit Lungenkrebs
bestimmt wird, und
wobei bevorzugter
das Referenzlevel das Level des RNA-Moleküls ist, das empirisch durch Messung einer Anzahl von Referenzblutproben von gesunden Probanden bestimmt wird, und wobei das Level des RNA-Moleküls oberhalb des Referenzlevels anzeigt, dass der Patient an Lungenkrebs erkrankt ist, und/oder
das Referenzlevel das Level des RNA-Moleküls ist, das empirisch durch Messung einer Anzahl von Referenzblutproben von Probanden mit Lungenkrebs bestimmt wird, und wobei das mit dem Referenzlevel vergleichbare Level des RNA-Moleküls anzeigt, dass der Patient an Lungenkrebs erkrankt ist.

3. Verfahren zur Überwachung des Verlaufs von Lungenkrebs bei einem Patienten, das den folgenden Schritt umfasst:
Bestimmen des Levels eines RNA-Moleküls in einer Blutprobe eines Patienten, wobei das RNA-Molekül eine Nukleotidsequenz gemäß SEQ ID NO: 1 umfasst.

4. Verfahren nach Anspruch 3, wobei die Überwachung das Bestimmen des Levels des RNA-Moleküls in einer Blutprobe, die von einem Patienten zu einem ersten Zeitpunkt erhalten wurde, und das Bestimmen des Levels in mindestens einer weiteren Blutprobe, die von dem (gleichen) Patienten zu einem späteren Zeitpunkt erhalten wurde, und den Vergleich der zu den verschiedenen Zeitpunkten bestimmten Levels umfasst.

5. Verfahren nach Anspruch 4, wobei das Level des RNA-Moleküls, das
(i) im Laufe der Zeit ansteigt, anzeigt, dass der Patient Lungenkrebs entwickelt hat oder dass sich der Lungenkrebs bei dem Patienten verschlimmert,
(ii) sich im Laufe der Zeit nicht ändert, anzeigt, dass der Patient stabil ist oder dass der Lungenkrebs bei dem Patienten nicht fortschreitet, oder
(iii) im Laufe der Zeit abnimmt, anzeigt, dass sich der Lungenkrebs bei dem Patienten verbessert.

6. Verfahren zur Bestimmung, ob ein Patient auf eine therapeutische Behandlung von Lungenkrebs anspricht, umfassend den Schritt:
Bestimmen des Levels eines RNA-Moleküls in einer Blutprobe eines Patienten, wobei das RNA-Molekül eine Nukleotidsequenz gemäß SEQ ID NO: 1 umfasst.

7. Verfahren nach Anspruch 6, wobei der Patient ein Patient ist, dem mindestens einmal ein in der therapeutischen Behandlung zu verwendendes Medikament und/oder eine andere Form der therapeutischen Behandlung verabreicht worden ist oder verabreicht wurde.

8. Verfahren zur Bestimmung des Risikos für einen Lungenkrebsrückfall bei einem Patienten, umfassend den Schritt:
Bestimmen des Levels eines RNA-Moleküls in einer Blutprobe eines Patienten, wobei das RNA-Molekül eine Nukleotidsequenz gemäß SEQ ID NO: 1 umfasst.

9. Verfahren nach Anspruch 8, wobei der Patient eine therapeutische Behandlung von Lungenkrebs erhalten hat, wobei die Behandlung vorzugsweise die Verabreichung eines Arzneimittels gegen Lungenkrebs und/oder
eine andere Form der therapeutischen Behandlung von Lungenkrebs umfasste.

10. Verfahren zum Nachweis einer minimalen Resterkrankung bei einem Patienten mit Lungenkrebs, umfassend den Schritt:
Bestimmen des Levels eines RNA-Moleküls in einer Blutprobe eines Patienten, wobei das RNA-Molekül eine Nukleotidsequenz gemäß SEQ ID NO: 1 umfasst.

11. Verfahren nach Anspruch 10, wobei der Patient eine therapeutische Behandlung von Lungenkrebs erhält oder erhalten hat, wobei die Behandlung vorzugsweise die Verabreichung
eines Arzneimittels gegen Lungenkrebs, und/oder
eine andere Form der therapeutischen Behandlung von Lungenkrebs umfasst oder umfasste.

12. Verfahren nach einem der Ansprüche 1 bis 11, wobei der Lungenkrebs ein nichtkleinzelliges Lungenkarzinom (NSCLC) ist.

13. Verfahren nach einem der Ansprüche 1 bis 12, wobei die Blutprobe Vollblut oder eine Blutfraktion ist.

14. Verwendung eines RNA-Moleküls zur Diagnose von Lungenkrebs bei einem Patienten, zur Überwachung des Verlaufs von Lungenkrebs bei einem Patienten, zur Bestimmung, ob ein Patient auf eine therapeutische Behandlung von Lungenkrebs anspricht, zur Bestimmung des Risikos für einen Lungenkrebsrückfall bei einem Patienten und/oder zum Nachweis einer minimalen Restkrankheit bei einem Patienten mit Lungenkrebs, wobei das RNA-Molekül eine Nukleotidsequenz gemäß SEQ ID NO: 1 umfasst.

15. Verwendung eines Kits zur Diagnose von Lungenkrebs bei einem Patienten, zur Überwachung des Verlaufs von Lungenkrebs bei einem Patienten, zur Bestimmung, ob ein Patient auf eine therapeutische Behandlung von Lungenkrebs anspricht, zur Bestimmung des Risikos für einen Lungenkrebsrückfall bei einem Patienten und/oder zum Nachweis einer minimalen Resterkrankung bei einem Patienten mit Lungenkrebs, wobei das Kit umfasst:
Mittel zum Bestimmen des Levels eines RNA-Moleküls in einer Blutprobe eines Patienten, wobei das RNA-Molekül eine Nukleotidsequenz gemäß SEQ ID NO: 1 umfasst.

## Revendications

1. Méthode de diagnostic du cancer du poumon chez un patient comprenant l'étape de :
détermination du taux d'une molécule d'ARN dans un échantillon de sang provenant d'un patient, ladite molécule d'ARN comprenant une séquence nucléotidique selon SEQ ID NO : 1.

2. Méthode selon la revendication 1,
ledit taux de la molécule d'ARN étant comparé à un taux de référence de ladite molécule d'ARN,
de préférence ledit taux de référence étant le taux de la molécule d'ARN déterminé de manière empirique par la mesure d'un nombre d'échantillons de sang de référence provenant de sujets sains, et/ou
de sujets atteints du cancer du poumon, et
plus préférablement
ledit taux de référence étant le taux de la molécule d'ARN déterminé de manière empirique par la mesure d'un nombre d'échantillons de sang de référence provenant de sujets sains, et ledit taux de la molécule d'ARN supérieur au taux de référence indiquant que le patient souffre du cancer du poumon, et/ou
ledit taux de référence étant le taux de la molécule d'ARN déterminé de manière empirique par la mesure d'un nombre d'échantillons de sang de référence provenant de sujets atteints du cancer du poumon, et ledit taux de la molécule d'ARN comparable au taux de référence indiquant que le patient souffre du cancer du poumon.

3. Méthode de surveillance de l'évolution du cancer du poumon chez un patient comprenant l'étape de :
détermination du taux d'une molécule d'ARN dans un échantillon de sang provenant d'un patient, ladite molécule d'ARN comprenant une séquence nucléotidique selon SEQ ID NO : 1.

4. Méthode selon la revendication 3, ladite surveillance comprenant la détermination du taux de la molécule d'ARN dans un échantillon de sang prélevé chez un patient à un premier moment dans le temps et dans au moins un autre échantillon de sang prélevé chez le (même) patient à un moment ultérieur dans le temps et la comparaison desdits taux déterminés aux différents moments dans le temps.

5. Méthode selon la revendication 4, ledit taux de la molécule d'ARN qui
(i) augmente au fil du temps indiquant que le patient a développé le cancer du poumon ou que le cancer du poumon est en train de s'aggraver chez le patient,
(ii) ne change pas au fil du temps indiquant que le patient est stable ou que le cancer du poumon n'est pas en train de progresser chez le patient, ou
(iii) diminue au fil du temps indiquant que le cancer du poumon est en train de s'améliorer chez le patient.

6. Méthode permettant de déterminer si un patient répond à un traitement thérapeutique du cancer du poumon comprenant l'étape de :
détermination du taux d'une molécule d'ARN dans un échantillon de sang provenant d'un patient, ladite molécule d'ARN comprenant une séquence nucléotidique selon SEQ ID NO : 1.

7. Méthode selon la revendication 6, ledit patient étant un patient chez lequel au moins une fois
un médicament à utiliser dans ledit traitement thérapeutique, et/ou
une autre forme de traitement thérapeutique
a été administré ou avait été administré.

8. Méthode de détermination du risque d'une rechute du cancer du poumon chez un patient comprenant l'étape de :
détermination du taux d'une molécule d'ARN dans un échantillon de sang provenant d'un patient, ladite molécule d'ARN comprenant une séquence nucléotidique selon SEQ ID NO : 1.

9. Méthode selon la revendication 8, ledit patient ayant reçu un traitement thérapeutique du cancer du poumon, ledit traitement ayant impliqué de préférence l'administration
d'un médicament pour le cancer du poumon, et/ou
d'une autre forme de traitement thérapeutique du cancer du poumon.

10. Méthode de détection d'une maladie résiduelle minimale chez un patient atteint du cancer du poumon comprenant l'étape de :
détermination du taux d'une molécule d'ARN dans un échantillon de sang provenant d'un patient, ladite molécule d'ARN comprenant une séquence nucléotidique selon SEQ ID NO : 1.

11. Méthode selon la revendication 10, ledit patient ayant reçu récemment ou ayant reçu par le passé un traitement thérapeutique du traitement du cancer, ledit traitement impliquant ou ayant impliqué de préférence l'administration
d'un médicament pour le cancer du poumon, et/ou
d'une autre forme de traitement thérapeutique du cancer du poumon.

12. Méthode selon l'une quelconque des revendications 1 à 11, ledit cancer du poumon étant un carcinome pulmonaire non à petites cellules (NSCLC).

13. Méthode selon l'une quelconque des revendications 1 à 12, ledit échantillon de sang étant du sang entier ou une fraction de sang.

14. Utilisation d'une molécule d'ARN pour diagnostiquer le cancer du poumon chez un patient, surveiller l'évolution du cancer du poumon chez un patient, déterminer si un patient répond à un traitement thérapeutique du cancer du poumon, déterminer le risque d'une rechute du cancer du poumon chez un patient, et/ou détecter une maladie résiduelle minimale chez un patient atteint du cancer du poumon, ladite molécule d'ARN comprenant une séquence nucléotidique selon SEQ ID NO : 1.

15. Utilisation d'un kit permettant de diagnostiquer le cancer du poumon chez un patient, surveiller l'évolution du cancer du poumon chez un patient, déterminer si un patient répond à un traitement thérapeutique du cancer du poumon, déterminer le risque d'une rechute du cancer du poumon chez un patient, et/ou détecter une maladie résiduelle minimale chez un patient atteint du cancer du poumon, ledit kit comprenant :
un moyen pour déterminer le taux d'une molécule d'ARN dans un échantillon de sang provenant d'un patient, ladite molécule d'ARN comprenant une séquence nucléotidique selon SEQ ID NO : 1.
